(19) Europäisches Patentamt
European Patent Office
Office européen des brevets

(11) EP 2 435 031 B1

(12) # FASCICULE DE BREVET EUROPEEN

(45) Date de publication et mention de la délivrance du brevet:
**16.09.2015 Bulletin 2015/38**

(21) Numéro de dépôt: **10727016.7**

(22) Date de dépôt: **28.05.2010**

(51) Int Cl.:
***A61K 9/51*** *(2006.01)* ***A61K 33/24*** *(2006.01)*
***A61P 35/00*** *(2006.01)*

(86) Numéro de dépôt international:
**PCT/FR2010/000401**

(87) Numéro de publication internationale:
**WO 2010/136676 (02.12.2010 Gazette 2010/48)**

(54) **Formulations à compartiments multiples à base de molécules ou macromolécules amphiphiles fonctionnelles**

Funktionelle amphipile Molekül- oder Makromolekülformulierungen mit mehreren Unterteilungen

Functional amphiphilic molecule or macromolecule formulations with multiple compartments

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **29.05.2009 FR 0902607**

(43) Date de publication de la demande:
**04.04.2012 Bulletin 2012/14**

(73) Titulaires:
- **Université de Bordeaux**
  **33000 Bordeaux (FR)**
- **Université d'Aix Marseille**
  **13284 Marseille Cedex 07 (FR)**

(72) Inventeurs:
- **BARTHELEMY, Philippe**
  **F-33700 Merignac (FR)**
- **KHIATI, Salim**
  **Bethesda, MD 20814 (FR)**
- **CAMPLO, Michel**
  **F-13007 Marseille (FR)**

(74) Mandataire: **Santarelli**
**49, avenue des Champs-Elysées**
**75008 Paris (FR)**

(56) Documents cités:
**WO-A1-2005/116043 WO-A2-2009/098404**
**US-A1- 2003 219 384 US-A1- 2008 089 836**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne de nouvelles formulations à compartiments multiples à base de molécules ou macromolécules amphiphiles fonctionnelles selon la revendication 1 pour le transport ou la vectorisation d'au moins un agent thérapeutique, en particulier un agent anti-tumoral ainsi que leur procédé de préparation, et leurs utilisations.

Parmi les agents anti-tumoraux, le cis-platine est un agent anti-tumoral largement utilisé, notamment pour le traitement de tumeurs solides. Cependant, son utilisation se trouve limitée par sa toxicité ainsi que l'apparition d'une résistance acquise.

Pour pallier ces inconvénients, différentes formulations ont été proposées dans l'art antérieur: par exemple, le brevet US 5 178 876 décrit des dérivés de platine sous forme de complexe hydrophobe destinés à une encapsulation dans des liposomes.

Le brevet US 6 001 817 décrit des compostions contenant du cis-platine et un vecteur comprenant au moins un nucléoside ou déoxynucléoside.

Le brevet US 7 908 160 concerne des dérivés de cis-platine liés à des ligands, dont l'activité est réversible en fonction de la liaison au ligand.

La demande WO01/32139 décrit des compositions de cis-platine encapsulé dans des nanoparticules lipidiques obtenues par cycles répétés de chauffage et congélation, à base de lipides naturels chargés négativement, en particulier la dioléylphosphatidylsérine. Il est indiqué dans cette demande que le cis-platine forme, dans l'eau, des agrégats chargés positivement présentant une solubilité dans plus élevée que les espèces non chargées, ce qui permet leur interaction avec les membranes lipidiques chargées négativement et la réorganisation des membranes lipidiques autour des agrégats de cis-platine.

La demande US-A-2008/089836 décrit des nanoparticules revêtues d'une bicouche de molécules formée à partir de:

(a) une couche de molécules liant la surface, en contact direct avec la nanoparticule, comprenant:

(i) un motif hydrophobe, et
(ii) un motif liant qui a une affinité pour la nanoparticule et

(b une couche de molécules amphiphiles, dans lesquelles la couche (a) de molécules liant la surface et la couche (b) de molécules amphiphiles sont liées par des interactions hydrophobes.

La demande WO-A-2009/098404 (citée à titre d'état de la technique relevant de l'article 54(3) CBE) divulgue un procédé de préparation de nanoparticules caractérisées en ce qu'elles sont constituées d'un coeur riche en agent thérapeutique, de préférence un agent anti-tumoral, entouré par une ou plusieurs couches lipidiques constituées d'un composé amphiphile fonctionnel et leur utilisation pour le transport ou la vectorisation d'agents thérapeutiques en particulier d'agent anti-tumoraux.

Cependant, il existe encore un besoin pour résoudre les problèmes liés à la vectorisation des agents thérapeutiques, en particulier les agents anti-tumoraux.

Notamment, il est recherché un moyen de permettre un transport des agents thérapeutiques (notamment le cis-platine et/ou ses dérivés) rapidement à l'intérieur des cellules tumorales avec une activité pharmacologique élevée, tout en préservant les cellules saines, c'est-à-dire en diminuant la toxicité neurologique, rénale, auditive, digestive, etc., en limitant simultanément les phénomènes d'apparition de résistance à cet agent thérapeutique.

Il est également recherché de fournir un vecteur présentant une stabilité dans le temps suffisante pour éviter la libération précoce de l'agent thérapeutique et les inconvénients liés à la présence de l'agent thérapeutique libre dans le milieu biologique, notamment en termes de perte d'activité et de toxicité.

Par ailleurs, la possibilité d'encapsuler un ou plusieurs agents thérapeutiques dans une même formulation dans différents compartiments d'une même formulation présente l'intérêt de permettre une délivrance simultanée ou échelonnée de cet (ces) agent(s) sur une même cible, chacun, des compartiments pouvant servir de réservoir.

On a maintenant trouvé que des formulations à compartiments multiples, formées à partir de molécules ou macromolécules amphiphiles fonctionnelles, présentaient des propriétés de stabilité améliorées, en particulier à 37°C, permettant une vectorisation prolongée dans le temps desdits agents thérapeutiques et permettaient la délivrance intracellulaire efficace et rapide d'agents thérapeutiques.

L'invention a donc pour objet, selon un premier aspect, une formulation à compartiments multiples sous forme de nanoparticule ayant un diamètre moyen d'environ 1 à 200 nm constituée d'un coeur solide contenant un agent thérapeutique, entouré par au moins deux couches lipidiques de polarité différente formées à partir de molécules ou macromolécules amphiphiles fonctionnelles, dans laquelle chaque couche lipidique est constituée d'au moins un composé amphiphile fonctionnel de formule (I) tel que défini ci-dessous.

Par « nanoparticule », on entend une particule ayant un diamètre moyen d'environ 1 à 200 nm, de préférence de 25 à 150 nm.

Dans la suite de la description, on entend par « nanoparticule selon l'invention » ou « nanoparticule à compartiments multiples » une formulation à compartiments multiples sous forme de nanoparticule constituée d'un coeur solide contenant un agent thérapeutique, entouré par au moins deux couches lipidiques de polarité différente formées à partir de molécules ou macromolécules amphiphiles fonctionnelles, dans laquelle chaque couche lipidique est constituée d'au moins un composé amphiphile fonctionnel de formule (I) tel que défini ci-dessous.

Avantageusement, chaque couche lipidique constitue un compartiment susceptible de comporter un agent thérapeutique identique ou différent de celui présent dans le coeur.

Selon un aspect avantageux, les formulations à compartiments multiples selon l'invention se forment à partir desdites couches lipidiques de polarité différente en présence du ou des agent(s) thérapeutiques et non à partir d'une particule pré-formée, ce qui permet une encapsulation «à la carte » du principe actif dans le ou les compartiment(s) voulu(s), en fonction de l'activité souhaitée.

Cette structure particulière confère aux nanoparticules à compartiments multiples selon l'invention une stabilité (durée de vie) compatible avec la délivrance d'un agent thérapeutique, et permet leur désagrégation après libération de cet agent thérapeutique.

De préférence, la première couche lipidique sera constituée d'un ou plusieurs lipide(s) anionique(s) et la seconde couche lipidique sera constituée d'un ou plusieurs lipide(s) cationique(s).

Par «de polarité différente », on entend que chaque couche lipidique successive entourant le coeur est constituée de lipides différents de la précédente et que chaque couche a une charge de surface globale soit négative (constituée de lipides anioniques) soit positive (constituée de lipides cationiques), soit neutre (constituée de lipides neutres). Par exemple, une première couche lipidique peut être formée de lipides anioniques et portera une charge de surface négative, alors que la deuxième couche lipidique peut être formée de lipides cationiques et portera une charge de surface positive.

La charge de surface de chacune des couches est mesurable par leur potentiel zeta, par exemple, selon la technique décrite dans Andrea Mayer et al. Toxicology, 2009, 258, 139-147 ou K. Furusawa et K. Uchiyama, 1988, 140, 217-226.

Selon un aspect avantageux, les nanoparticules à compartiments multiples selon l'invention peuvent comprendre une alternance de couches lipidiques anioniques et cationiques et une couche externe constituée d'un ou plusieurs lipides neutre(s).

Selon l'invention, chaque couche lipidique est constituée d'au moins un composé amphiphile fonctionnel de formule (I)

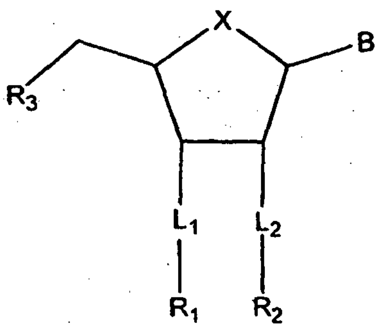

(I)

dans laquelle

- X représente un atome d'oxygène, de soufre ou un groupe méthylène,
- B représente une base purique ou pyrimidique telle que uracile, adénine, guanine, cytosine, thymine, hypoxanthine, ou leurs dérivés, ou encore une base hétérocylique mono-ou bi-cyclique non naturelle dont chaque cycle comporte 4 à 7 chaînons, éventuellement substituée ;
- $L_1$ et $L_2$, identiques ou différents, représentent l'hydrogène, un groupe oxycarbonyl -O-C(O)-, un groupe thiocarbamate -O-C(S)-NH-, un groupe carbonate -O-C(O)-O-, un groupe carbamate -O-C(O)-NH-, un atome d'oxygène, un groupe phosphate, un groupe phosphonate ou un groupe hétéroaryle comprenant 1 à 4 atomes d'azote, substitué ou non substitué par une chaîne hydrocarbonée, saturée ou insaturée, linéaire ou ramifiée en $C_2$-$C_{30}$,
ou encore, $L_1$ et $L_2$, ensemble, forment un groupement cétal de formule

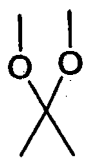

ou encore $L_1$ ou $L_2$ représente l'hydrogène, et l'autre représente un groupe hydroxy ou un groupe hétéroaryle comprenant 1 à 4 atomes d'azote, non substitué ou substitué par une chaîne alkyle linéaire ou ramifiée en $C_2$-$C_{30}$:

- $R_1$ et $R_2$, identiques ou différents, représentent

  - une chaîne hydrocarbonée linéaire ou ramifiée en $C_2$-$C_{30}$, de préférence en $C_6$-$C_{25}$, notamment en $C_8$-$C_{25}$, saturée ou partiellement insaturée, éventuellement totalement ou partiellement fluorée, non substituée ou substituée sur le carbone d'extrémité de chaîne par un atome de fluor ou par un ester ou un éther benzylique ou naphtylique, ou
  - une chaîne diacyle dans laquelle chaque chaîne acyle en $C_2$-$C_{30}$, ou
  - un groupe diacylglycérol, sphingosine ou céramide, ou
  - lorsque $L_1$ ou $L_2$ représente l'hydrogène, et l'autre représente un groupe hydroxy ou un groupe hétéroaryle comprenant 1 à 4 atomes d'azote, $R_1$ et $R_2$ n'existent pas;

- $R_3$ représente

  - un groupement hydroxy, amino, phosphate, phosphonate, phosphatidylcholine, O-alkyl phosphatidylcholine, thiophosphate, phosphonium, $NH_2$-$R_4$, $NHR_4R_5$ ou $NR_4R_5R_6$ dans lesquels $R_4$, $R_5$ et $R_6$, identiques ou différents, représentent un atome d'hydrogène ou une chaîne alkyle linéaire ou ramifiée en $C_1$-$C_5$ ou hydroxyalkyle linéaire ou ramifié en $C_1$-$C_5$, ou
  - une chaîne alkyle linéaire ou ramifiée en $C_2$-$C_{30}$ éventuellement substituée par un groupe hydroxy , ou
  - un reste cyclodextrine, ou
  - un reste

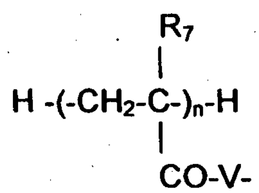

    dans lequel V représente une liaison -O-,-S-, ou -NH-, $R_7$ représente H ou $CH_3$, et n= 1 à 500, ou un groupe $-(CH_2)_n$-V-$R_8$, dans lequel Re représente un alkyle en $C_2$-$C_{30}$, et n=1 à 500, ou
  - un groupe hétéroaryle renfermant 1 à 4 atomes d'azote, non substitué ou substitué par un alkyle en $C_2$-$C_{30}$, ou par un groupe $(CH_2)_m$-O-$(CH_2)_p$-$R_9$ dans lequel m = 1 à 6 et p = 0 à 10 et $R_9$ représente un groupe cétal cyclique renfermant 5 à 7 atomes de carbone, non substitué ou substitué par au moins un alkyle linéaire ou ramifié en $C_2$-$C_{30}$ ou par un reste stérol, ou encore
  - $R_3$ est lié par liaison covalente à un autre substituant $R_3$, identique ou différent, d'un autre composé de formule (I), identique ou différent, pour former un composé de formule (I) sous forme de dimère,

et chaque couche lipidique a une polarité différente de celle de la précédente.

La charge des composés de formule (I) est déterminée par les groupements polaires qu'ils contiennent, ceux-ci étant essentiellement présents dans ou constitués par les substituant $L_1$, $L_2$ et/ou $R_3$.

Des composés de formule (I) anioniques utilisables pour préparer la première couche lipidique peuvent être, par exemple, choisis parmi des nucléolipides anioniques tels que des composés de formule (I) dans laquelle $L_1$, $L_2$ et/ou $R_3$ représentent un groupement chargé négativement tel que, par exemple, un groupement phosphate, phosphonate, carboxylate, sulfate, etc.., éventuellement substitué.

Des composés de formule (I) cationiques utilisables pour préparer la première couche lipidique peuvent être, par exemple, choisis parmi des nucléolipides cationiques tels que des composés de formule (I) dans laquelle $L_1$, $L_2$ et/ou $R_3$ représentent un groupement chargé positivement tel que, par exemple un groupement ammonium, phosphonium, imidazolium, etc., éventuellement substitué.

La charge de ces groupements polaires peut également varier en fonction du pKa de ces groupements, par

exemple lorsqu'il s'agit d'un groupement amine, imidazole, phosphate, etc

Par « agent thérapeutique », on entend, par exemple, une molécule naturelle ou synthétique utilisée pour la prévention ou le traitement d'une pathologie ou la restauration d'une fonction biologique, in vitro ou in vivo, en particulier chez l'animal, y compris l'être humain, ou encore sur des cellules isolées, à l'exception des acides nucléiques ou de leurs fragments.

Une telle molécule peut être choisie, par exemple, parmi les principes actifs de médicaments, en particulier parmi les agents anti-tumoraux tels que, par exemple :

- les complexes de platine, parmi lesquels on peut notamment citer le cis-platine, le carboplatine, l'oxaliplatine, le nédaplatine, le lobaplatine, etc.,ou
- le ruthénium capable de se lier à des complexes de platines, ou encore
- les complexes inorganiques sans platine à base de ruthénium II et/ou III, de titane, par exemple le dichlorure de titanocène, ou de gallium, par exemple les sels de gallium tels que le nitrate de gallium, le chlorure de gallium, le KP46, ou
- les dérivés du fer, tels que, par exemple, les sels de ferrocenium, les analogues nucléosidiques contenant du fer, les complexes de fer (II) contenant des ligands pyridyl-pentadéntate, ou
- les dérivés du cobalt, tels que, par exemple, les complexes d'hexacarbonyl-dicobalt, les complexes d'alkyne-cobalt, le complexe de Co(III) contenant un ligand azote motarde, ou
- les dérivés de l'or tels que, par exemple, l'Auranofin, les complexes d'or (I), (III) et (III), l'aurothioglucose, etc.

Avantageusement, la formulation à compartiments multiples selon l'invention permet d'encapsuler ces molécules et d'assurer leur délivrance intracellulaire tout en limitant les phénomènes de résistance acquise à ces composés.

Les complexes de platine, en particulier le cis-platine, sont des agents thérapeutiques préférés aux fins de l'invention.

Des complexes inorganiques à base de ruthénium II et/ou III, peuvent être, par exemple, les complexes dénommés NAMI-A, RAPTA-C, KP1019. De tels complexes non platinés sont décrits dans Ott I. et Gust R., Arch. Pharm. Chem. Life Sci. 2007, 340, 117-126 ; Reedijk J., Curr Opin Chem Biol., 1999, 3, 236-40 ; Haimei Chen et al., J. Am. Chem. Soc., 2003, 125, 173-186.

Des analogues nucléosidiques contenant du fer sont décrits dans Schlawe D. et al, Angew. Chem. Int. Ed., 2004, 1731-1734.

De manière avantageuse, on a trouvé que les structures moléculaires et/ou macromoléculaires qui constituent les composés de formule (I), comprenant au moins un ligand de l'agent thérapeutique (nucléobase, nucléoside, nucléoside modifié, nucléotides, oligonucléotide, hétérocycle, etc) repésenté par le substituant B, et présentant un caractère amphiphile du fait de la présence d'au moins une partie hydrophile (phosphate, carboxylate, etc), et d'au moins une partie hydrophobe (segments hydrophobes monocaténaires, bicaténaires et parties polaires dérivées de synthons d'origine biologique, etc), permettaient de former avec l'agent thérapeutique des nanoparticules stables.

Par la combinaison des propriétés amphiphiles des composés de formule (I), de la présence de ligands de l'agent thérapeutique (principe actif) dans ces composés et des éventuelles interactions électrostatiques entre les agents thérapeutiques et ces composés, les nanoparticules ainsi obtenues présentent une structure permettant une délivrance intra-cellulaire efficace et rapide des principes actifs encapsulés, en particulier des agents anti-tumoraux.

Sans vouloir lier l'invention à une théorie, on peut émettre l'hypothèse selon laquelle les interactions intermoléculaires des composés de formule (I) induisent une augmentation des forces de cohésion à la surface des nanoparticules, ce qui se traduit par une plus grande stabilité dans le temps, dans les conditions d'utilisation.

La structure à compartiments multiples des nanoparticules selon l'invention, à base de couches lipidiques multiples ayant une polarité modulable, leur confère de nombreux avantages, en particulier :

- une stabilité accrue, notamment en milieu biologique,
- la modulation du potentiel de surface (potentiel zeta) en fonction de leur efficacité dans l'utilisation envisagée,
- leur fonctionnalisation (introduction d'une fonctionnalité, agent de ciblage, etc),
- l'incorporation de différents agents thérapeutiques.

Avantageusement, lesdites nanoparticules présentent également une durée de vie compatible avec leur utilisation comme vecteur d'agent thérapeutique.

Dans la formule (I) ci-dessus, n est avantageusement compris entre 1 et 500, de préférence compris entre 1 et 100, notamment compris entre 1 et 50, tout particulièrement compris entre 1 et 10.

Par « alkyle linéaire ou ramifié en $C_1$-$C_5$ », on entend par exemple un radical méthyle, éthyle, propyle, i-propyle, n-butyle, i-butyle, tert-butyle, de préférence méthyle ou éthyle.

Egalement, dans la formule (I) ci-dessus, la base purique ou pyrimidique, ou la base hétérocyclique non naturelle peut être substituée par au moins un substituant choisi, par exemple, parmi un halogène, un groupe amino, un groupe carboxy , un groupe carbonyl, un groupe carbonylamino, un groupe hydroxy, azido, cyano, alkyle, cycloalkyl, perfluoroalkyl, alkyloxy (par exemple, méthoxy), oxycarbonyl, vinyl, ethynyl, propynyl, acyl etc.

On entend par « base hétérocyclique non naturelle » une base autre que uracile, adénine, guanine, cytosine, thymine ou hypoxanthine, qui n'existe pas dans la nature.

Par « groupe hétéroaryle renfermant 1 à 4 atomes d'azote », on entend un groupement carbocyclique monocyclique ou bycyclique, aromatique ou partiellement insaturé, renfermant 5 à 12 atomes, interrompu par 1 à 4 atomes d'azote, en particulier les groupes pyrazole, triazole, tétrazole ou imidazole.

Pour la préparation des composés de formule (I), on peut se référer à la demande WO 2005/116043, qui décrit différentes voies d'accès à ce type de composés (voir notamment p. 8-17 et exemples).

L'invention concerne également, selon un aspect ultérieur, un procédé de préparation d'une formulation à compartiments multiples sous forme de nanoparticule solide constituée d'un coeur contenant un agent thérapeutique, entouré par au moins deux couches lipidiques de polarité différente formées à partir de molécules ou macromolécules amphiphiles fonctionnelles telle que définie dans la revendication 1, dans laquelle chaque couche lipidique constitue un compartiment susceptible de comporter un agent thérapeutique identique à ou différent de celui présent dans le coeur, comprenant les étapes suivantes :

a) préparer un mélange d'au moins un composé amphiphile fonctionnel de formule (I) tel que défini ci-dessus, et d'un agent thérapeutique,
b) soumettre ledit mélange à des cycles répétés de chauffage et congélation, de manière à obtenir des nanoparticules contenant ledit agent thérapeutique, et
c) récupérer les nanoparticules contenant ledit agent thérapeutique ainsi obtenues,
d) mettre en présence lesdites nanoparticule avec au moins un composé amphiphile fonctionnel de formule (I) tel que défini ci-dessus, ayant une polarité différente de celui mis en oeuvre dans l'étape a), et
e) récupérer les nanoparticules multi-compartiments ainsi obtenues.

De préférence, l'agent thérapeutique est un agent anti-tumoral, en particulier un complexe de platine, en particulier le cis-platine.

Avantageusement, les étapes du procédé pourront être répétées le nombre de fois nécessaire pour obtenir le nombre de couches lipidiques voulu.

De manière optionnelle, une étape supplémentaire consistant en la formation d'une couche lipidique neutre constituée d'au moins d'au moins une molécule ou macromolécule amphiphile fonctionnelle, en particulier un composé amphiphile fonctionnel de formule (I) telle que définie ci-dessus, ladite molécule ou ledit composé de formule (I) étant neutre, pourra être effectuée entre l'étape d) et l'étape e).

Selon un aspect préféré, on utilisera dans l'étape a) et/ou l'étape d), en complément du composé amphiphile fonctionnel, au moins un colipide.

Par « colipide », on entend un composé utilisé en association avec le composé de formule (I), qui participe à l'élaboration de la structure de la ou des couche(s) lipidique(s) de la nanoparticule.

On utilisera, de préférence, un colipide zwitterionique.

Ledit colipide peut être, par exemple, choisi parmi la dioléylphosphatidylcholine (DOPC), la diotéylphosphatidyluridine-phosphatidylcholine (DOUPC) ou la dioléylphosphatidyléthanolamine (DOPE).

Ces composés peuvent jouer le rôle de colipide lorsqu'ils sont utilisés en mélange avec un composé de formule (I). Alternativement, ils peuvent être compris dans la formule (I), comme, par exemple, la dioléylphosphatidyluridinephosphatidylcholine (DOUPC). Dans ce cas, ils joueront, soit le rôle de composé de formule (I), soit, en combinaison avec un autre composé de formule (I), le rôle de colipide.

Selon un autre aspect avantageux, on introduit à l'étape d) un agent thérapeutique identique ou différent de celui utilisé dans l'étape a).

De préférence, le ou les composé(s) amphiphile(s) fonctionnel(s) de formule (I) utilisé(s) dans l'étape a) est (sont) anionique(s) et le ou les composé(s) amphiphile(s) fonctionnel(s) de formule (I) utilisé(s) dans l'étape d) est (sont) cationique(s).

Avantageusement, on utilisera un composé amphiphile fonctionnel de formule (I) neutre pour constituer la couche lipidique la plus externe , qui pourra être effectuée entre l'étape d) et l'étape e).

Plus particulièrement, le procédé de préparation des formulations à compartiments multiples peut comprendre

les étapes mises en oeuvre dans les conditions générales suivantes, qui illustrent, par exemple, l'obtention d'une formulation à compartiments multiples sous forme de nanoparticule constituée d'un coeur solide contenant un agent thérapeutique, entouré par deux couches lipidiques de polarité différente formées à partir de composés de formule (I):

1) Formation de nanoparticules selon l'invention comprenant un coeur riche en agent thérapeutique et_une première couche lipidique

- un composé de formule (I) est mis en solution dans un solvant organique pour former un mélange lipidique, puis, le solvant est évaporé pour former un premier film lipidique;
- parallèlement, la quantité voulue d'agent thérapeutique, de préférence un agent anti-tumoral, est mise en solution dans de l'eau distillée ;
- le premier film lipidique est réhydraté dans la solution d'agent thérapeutique, de préférence un agent anti-tumoral. Une solution limpide est obtenue par sonification et chauffage ;
- la solution est refroidie rapidement, par exemple par immersion dans l'azote liquide. Ce cycle chauffage / refroidissement est effectué de préférence de 1 à 10 fois, en particulier de 5 à 10 fois, notamment 10 fois ;
- après sonification et centrifugation de la suspension obtenue, le surnageant est écarté et le culot remis en suspension ;
- après centrifugation, le culot est écarté et le surnageant est récupéré.

2) Formation de nanoparticules selon l'invention comprenant un coeur riche en agent thérapeutique et deux couches lipidiques de polarité différentes

- on prépare un second film lipidique à partir d'un composé de formule (I) de polarité différente de celle du composé de formule (I) utilisé dans la première partie du procédé ;
- on réhydrate le second film lipidique avec le surnageant précédemment récupéré,
- après sonification et centrifugation de la suspension obtenue, le surnageant est écarté et le culot remis en suspension ;
- après centrifugation, le culot est écarté et le surnageant est récupéré, contenant les nanoparticules à compartiments multiples comprenant deux couches lipidiques de polarité différente.

Avantageusement, on répète les étapes du procédé ci-dessus le nombre de fois nécessaire à l'obtention du nombre de couches lipidiques voulu.

De préférence, le nombre de couches lipidiques sera compris entre 2 et 6.

De manière optionnelle, une étape supplémentaire consistant en la formation d'une couche lipidique constituée d'au moins un composé amphiphile fonctionnel de formule (I) telle que définie plus haut, ledit composé de formule (I) étant neutre, pourra être effectuée lors de la 2$^{ème}$ partie du procédé, avant l'étape finale permettant la récupération des nanoparticules à compartiments multiples selon l'invention.

Selon un aspect préféré, on utilisera lors de la formation du mélange lipidique dans la première partie de la préparation décrite ci-dessus, ou dans sa deuxième partie, en complément du composé de formule (I), au moins un colipide, tel que défini plus haut.

Des formulations préférées selon l'invention sont celles dans lesquelles la première couche lipidique est anionique et la deuxième couche lipidique est cationique.

Le solvant organique peut être choisi, par exemple, parmi les solvants organiques usuels dans le domaine, tels que, par exemple, le chloroforme ou le dichlorométhane, un alcool tel que le méthanol ou l'éthanol, etc.

Le chauffage est effectué, de préférence, à une température de l'ordre de 20°C à 80°C, et le refroidissement à une température de l'ordre de-190°C (azote liquide) à 0°C (glace). Un cycle chauffage/refroidissement approprié peut, par exemple, être de 45°C pour le chauffage et de -78°C pour le refroidissement.

De préférence, l'agent thérapeutique est choisi parmi les complexes de platine (cis-platine, carboplatine, ...), le cis-platine étant particulièrement préféré, ou bien le ruthénium capable de se lier à des complexes de platines, ou encore les complexes inorganiques sans platine à base de ruthénium II ou III, de titane ,de gallium, de cobalt, de fer ou d'or mentionnés plus haut.

Le rapport molaire R du composé de formule (I) / agent thérapeutique peut être compris, par exemple, entre 0,01 à 50, en particulier R = 0,2.

Les nanoparticules obtenues peuvent être éventuellement extrudées sur filtre polycarbonate ayant, par exemple, un diamètre de pores de l'ordre de 100 ou 200 nm.

On obtient ainsi des nanoparticules à compartiments multiples qui sont constituées d'un coeur solide riche en agent thérapeutique (principe actif) entouré par au moins deux couches lipidiques de polarité différente constituées de composé amphiphile fonctionnel de formule (I) telle que définie plus haut, avec ou sans co-lipide.

Selon un aspect du procédé, ledit mélange lipidique contient uniquement au moins un composé de formule (I) et ne contient pas de colipide.

On utilisera de préférence l'agent thérapeutique à une concentration de l'ordre de 0,1 ng/mL à 10 mg/mL dans la phase aqueuse, de manière à ce que la délivrance intracellulaire du principe actif soit importante.

Des composés préférés de formule (I) utilisables pour former une couche lipidique sont ceux dans lesquels X représente l'oxygène.

Les composés de formule (I) dans lesquels B représente la thymine ou l'adénine sont également des composés préférés.

Les composés de formule (I) dans laquelle $L_1$, $L_2$ et/ou $R_3$ représentent un groupement chargé négativement tel que, par exemple, un groupement phosphate, phosphonate, carboxylate, sulfate, etc.., éventuellement substitués, sont des composés préférés pour obtenir une couche lipidique anionique.

Les composés de formule (I) dans laquelle dans lesquels $L_1$, $L_2$ et/ou $R_3$ représentent un groupement chargé positivement tel que, par exemple un groupement ammonium, phosphonium, imidazolium, etc.., éventuellement substitués, sont des composés préférés pour obtenir une couche lipidique cationique.

Selon un aspect préféré, l'invention concerne les nanoparticules à compartiments multiples telles que définies plus haut comprenant ces composés de formule (I) et un agent thérapeutique, en particulier un agent anti-tumoral, en particulier les complexes de platine (tels que, par exemple le cis-platine, le carboplatine, l'oxaliplatine, le nédaplatine, le lobaplatine,), ou le ruthénium capable de se lier à des complexes de platines, ou encore des complexes inorganiques sans platine à base de ruthénium, de titane, de gallium, de cobalt, de fer ou d'or mentionnés plus haut. Le cis-platine est un agent anti-tumoral préféré aux fins de l'invention.

Les composés de formule (I) peuvent également comporter des dérivés de base purique ou pyrimidique ayant une activité antinéoplasique, tels que, par exemple, l'aracytosine (AraC), le 5-fluorouracile (5-FU), l'lododéoxyuridine (IdU), la 2'-déoxy-2'-méthylidènecytidine (DMDC) ou la 5-chloro-6-azido-5,6-dihydro-2'-déoxyuridine.

L'invention a également pour objet l'utilisation des nanoparticules à compartiments multiples décrites ci-dessus, en tant qu'agent pour le transport ou la vectorisation d'agent thérapeutiques, en particulier d'agents anti-humoraux

En particulier, l'invention concerne l'utilisation des nanoparticules à compartiments multiples décrites ci-dessus, en tant qu'agent pour la délivrance intracellulaire d'agents thérapeutiques, en particulier d'agents anti-tumoraux.

L'invention concerne également l'utilisation des nanoparticules à compartiments multiples décrites ci-dessus, pour la préparation de médicaments anti-tumoraux.

L'invention concerne également les nanoparticules à compartiments multiples décrites ci-dessus, pour le traitement de maladies tumorales, en particulier de cancers, tels que, par exemple, les cancers de l'ovaire, du testicule, du colon, du col de l'utérus, du poumon, ou l'adénosarcome etc.

Lesdites nanoparticules à compartiments multiples sont susceptibles d'être obtenues par le procédé décrit plus haut.

L'invention concerne également les compositions pharmaceutiques comprenant des formulations à compartiments multiples sous forme de nanoparticule constituée d'un coeur solide contenant un agent thérapeutique, entouré par au moins deux couches lipidiques de polarité différente formées à partir de molécules ou macromolécules amphiphiles fonctionnelles (ou nanoparticules à compartiments multiples), telles que décrites plus haut, et un véhicule pharmaceutiquement acceptable.

L'invention est illustrée par les exemples ci-dessous.

L'ensemble des produits de départ proviennent de fournisseurs de produits chimiques (Aldrich, Alfa Aesar et Avanti Polar Lipid) et sont utilisés sans purification ultérieure. Les solvants ont été utilisés sans distillation supplémentaire. Les composés synthétisés ont été caractérisés à l'aide de méthodes analytiques spectroscopiques standard telles que les RMN $^{1}H$ à 300,13 MHz, $^{13}C$ à 75,46 MHz et $^{31}P$ à 121,49 MHz) et la spectroscopie de masse (Caractéristiques). Les déplacements chimiques ($\delta$) en RMN sont exprimés en ppm et relativement au TMS. Les constantes de couplage J en RMN du $^{1}H$ sont exprimées en Hz. Des plaques Merck RP-18 F254s ont été utilisées pour la chromatographie sur couche mince (CCM). De la silice SEPHADEX LH-20 (25-100 $\mu$m) a été utilisée pour les purifications par chromatographies quantitatives.

Les exemples ci-dessous, intitulés « Préparation » décrivent la préparation d'intermédiaires de synthèse utilisés pour préparer les composés de formule (I). La préparation des composés de formule (I) et l'étude des nanoparticules selon l'invention sont décrites ensuite dans les exemples de synthèse et les essais intitulés « Exemple ».

Préparation 1

5'- paratoluènesulfonylthymidine

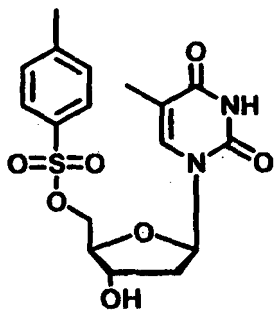

Dans un ballon bi-col sous atmosphère d'azote anhydre, on introduit 2 g de thymidine (8,26 mmol) en solution 0,1 M dans la pyridine anhydre. La solution est alors refroidie à 0°C et 3,935 g de chlorure d'acide paratoluène sulfonique (2,5 équivalents, 20,6 mmol) sont additionnés par petites fractions. On laisse revenir le milieu réactionnel à température ambiante, puis on agite pendant 10 h. La réaction est alors stoppée par ajout de 10 mL de méthanol, l'agitation est maintenue pendant 30 min. On ajoute au mélange 50 mL de $CH_2Cl_2$ puis on lave successivement par 20 mL d'une solution à 5% de $NaHCO_3$, 20 mL d'une solution saturée de NaCl et 20 mL d'une solution à 5% de $NaHCO_3$. Le solvant est éliminé sous pression réduite. Le composé attendu est obtenu pur par recristallisation dans le méthanol.
RF : 0,47 (AcOEt/MeOH 9/1)
Rendement : 75%
RMN $^1H$ (300,13 MHz, DMSO $d_6$) : δ 1,77 (s, 3H, $CH_3$), δ 2,11 (m, 2H, $CH_2$), δ 2,42 (s, 3H, CH3), δ 3,52 (t, *j* = 6 Hz, 4H, $CH_2$), δ 4,18 (m, 1H, CH), δ 4,25 (m, 3H, CH, $CH_2$), δ 5,42 (s, 1H, OH), δ 6,15 (t, *j* = 6 Hz, H, CH), δ 7,38 (s, 1 H, CH), δ 7,46 (s, 1 H, CH), δ 7,49 (s, 1 H, CH), δ 7,78 (s, 1H, CH), δ 7,81 (s, 1 H, CH), δ 11,28 (s, 1 H, NH).
RMN $^{13}C$ (75,47 MHz, DMSO $d_6$): δ 12,5 ($CH_3$),δ 21,6 ($CH_3$), δ 38,9 ($CH_2$), δ 70,4 ($CH_2$), δ 70,6 (CH),δ 83,7 (CH), δ 84,5 (CH), δ 110,3 (C), δ 128,1 (CH ar), δ 130,6 (2 CH ar), δ 132,6 (C ar), δ 136,4 (C ar), δ 145,6 (C), δ 150,8 (C=O), δ 164,1 (C=O).
SM Haute $[M+H]^+$ : 397,1

Préparation 2

2'-déoxy-5'-toluènesulfonyladénosine

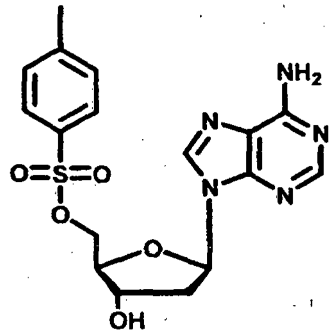

Dans un ballon bi-col sous atmosphère d'azote anhydre, on introduit 2 g de 2'-déoxyadénosine (8 mmol) en solution 0,1 M dans la pyridine anhydre. La solution est alors refroidie à 0°C et 3,793 g de chlorure d'acide paratoluène sulfonique (2,5 équivalents, 20 mmol) sont additionnés par petites fractions. On laisse revenir le milieu réactionnel à température ambiante, puis on agite pendant 10 h. La réaction est alors stoppée par ajout de 10 mL de méthanol, l'agitation est maintenue pendant 30 min. On ajoute au mélange 50 mL de $CH_2Cl_2$ puis on lave successivement par 20 mL d'une solution à 5% de $NaHCO_3$, 20 mL d'une solution saturée de NaCl et 20 mL d'une solution à 5% de $NaHCO_3$.

Le solvant est éliminé sous pression réduite. Le composé attendu est obtenu pur par recristallisation dans le méthanol. 2,1 g d'un produit blanc sont ainsi isolés.
RF: 0,37 (AcOEt/MeOH 9/1)
Rendement : 63%

Préparation 3

5'azido-5'-déoxythymidine

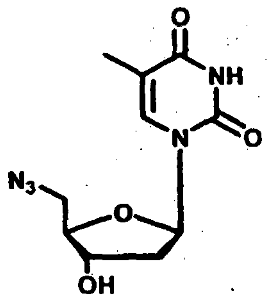

Dans un ballon bi-col muni d'un réfrigérant et sous atmosphère d'azote anhydre, on introduit 2 g de 5'-paratoluènesulfonylthymidine (5 mmol) telle que décrite dans la préparation 1 en solution 0,1 M dans le DMF. 1,3 g d'azidure de sodium (4 équivalents, 20 mmol) sont ajoutés. La solution est alors agitée et chauffée à 110°C pendant 10 h. Le mélange est refroidi à température ambiante. On ajoute au mélange 50 mL de $CH_2Cl_2$ puis on lave successivement par 2 fois 15 mL d'eau puis par 15 mL d'une solution aqueuse saturée de NaCl. La phase organique est séchée sur sulfate de sodium puis le solvant est éliminé sous pression réduite. Le composé attendu est obtenu pur par recristallisation dans le méthanol. On obtient ainsi 0,8 g d'un solide blanc.
RF: 0,47 (AcOEt/MeOH 9/1)
Rendement : 60%
SM $[M+H]^+$: 268.1

Préparation 4

5'-azido-5',2'-didéoxyadénosine

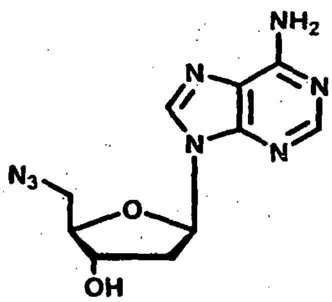

Dans un ballon bi-col muni d'un réfrigérant et sous atmosphère d'azote anhydre, on introduit 2 g de 2'-déoxy-5'-paratoluènesulfonyladénosine telle que décrite dans la préparation 2 (5 mmol) en solution 0,1 M dans le DMF. 1,3 g d'azidure de sodium (4 équivalents, 20 mmol) sont ajoutés. La solution est alors agitée et chauffée à 110°C pendant 10 h. Le mélange est refroidi à température ambiante. On ajoute au mélange 50 mL de $CH_2Cl_2$ puis on lave successivement par 2 fois 15 mL d'eau puis par 15 mL d'une solution aqueuse saturée de NaCl. La phase organique est séchée sur sulfate de sodium puis le solvant est éliminé sous pression réduite. Le composé attendu est obtenu pur par recristallisation dans le méthanol. On obtient ainsi 0,8 g d'un solide blanc.
RF : 0,37 (AcOEt/MeOH 9/1)

Rendement: 60%
SM Haute résolution $[M+H]^+$ : masse calculée : 277,1161, masse mesurée: 277,1157

Préparation 5

1-propargyloxyoctadécane

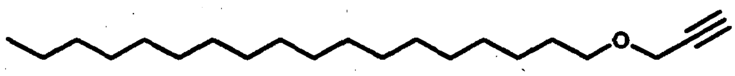

Dans un ballon propre et sec, sous atmosphère d'azote anhydre, sont introduits 673 mg d'alcool propargylique (12 mmol) en solution 0,5 M dans le DMF. La solution est alors refroidie à 0°C et 180 mg d'hydrure de sodium (0,625 équivalent, 7,5 mmol) sont additionnés par petites fractions. Le milieu réactionnel est laissé revenir à température ambiante. 2 g de 1-bromo-octadecane (0.5 équivalent), 6 mmol) sont ajoutés. L'agitation est maintenue pendant 5 h. La réaction est alors stoppée par ajout de 10 mL de méthanol et l'agitation est maintenue pendant 30 min. On ajoute au mélange 50 mL de $CH_2Cl_2$ puis on lave successivement par 2 fois 20 mL et 20 mL d'une solution saturée de NaCl. La phase organique est alors séchée sur $Na_2SO_4$ puis le solvant est éliminé sous pression réduite. Le composé attendu est obtenu pur après séparation sur colonne chromatographique (hexane). 1,2 g d'un produit blanc sont ainsi isolés.
RF: 0,82 (Hexane)
Rendement : 65 %
RMN $^1H$ (300,13 MHz, $CDCl_3$) : δ 0,90 (t, *j* = 6 Hz, 3H, $CH_3$), δ 1,28 (s, 30H, $CH_2$), δ 1,61 (m, 2H, $CH_2$),δ 2,43 (t, *j* = 3 Hz, 1H, CH), δ 3,53 (t, *j* = 6 Hz, 2H, $CH_2$), δ 4.15 (d, *j* = 3 Hz, 2H, $CH_2$).
RMN $^{13}C$ (75,47 MHz, $CDCl_3$) : RMN $^{13}C$ (75,47 MHz, $CDCl_3$) : δ 14,2 ($CH_2$), δ 22,7 ($CH_2$),δ 26,1 ($CH_2$),δ 29,4 ($CH_2$),δ 29,5 ($CH_2$), δ 29,6 ($CH_2$), δ 32,0 ($CH_2$), δ 58,0 ($CH_2$), δ 70,4 ($CH_2$), δ 74,1 (CH), δ 80,1 (C).

Préparation 6

1,12-propargyloxydodécane

12-propargyloxydodécan-1-ol

Dans un ballon propre et sec, sous atmosphère d'azote anhydre, on introduit 1 g de dodécan-1,12-diol (5 mmol) en solution 0,5 M dans le DMF. La solution est alors refroidie à 0°C et 360 mg d'hydrure d'hydrogène (3 équivalents, 15 mmol) sont additionnés par petites fractions. On laisse revenir le milieu réactionnel à température ambiante. 1.49 g de bromure de propargyle (2,5 équivalents, 12,5 mmol) sont ajoutés. L'agitation est maintenue pendant 5 h. La réaction est alors stoppée par ajout de 10 mL de méthanol, l'agitation est maintenue pendant 30 min. On ajoute au mélange 50 mL de $CH_2Cl_2$ puis on lave successivement par 2 fois 20 mL et 20 mL d'une solution saturée de NaCl. La phase organique est alors séchée sur $Na_2SO_4$ puis le solvant est éliminé sous pression réduite. Les produits obtenus sont alors séparés sur colonne chromatographique (Hex/ActEth 9/1). Deux produits sont isolés, à savoir 370 mg d'une huile brune correspondant au 1,12-Propargyloxydodécane et 430 mg d'un solide brun correspondant au 12-propargyloxydodécan-1-ol.

1,12-propargyloxydodécane

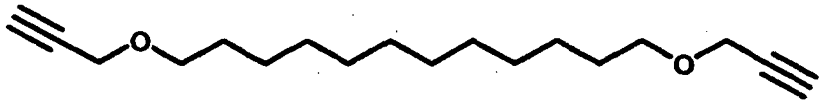

RF : 0,53 (Hexane/AcOtEt 9/1)
Rendement: 27 %
RMN $^1H$ (300,13 MHz, $CDCl_3$) : δ 1,31 (m, 16H, CH2), δ 1,61 (m, 4H, $CH_2$), δ 2,43 (t, *j* = 3 Hz, 1H, CH), δ 3,52 (t, *j* = 6 Hz, 4H, $CH_2$), δ 4,15 (d, *j* = 3 Hz, 4H, $CH_2$).
RMN $^{13}C$ (75,47 MHz, $CDCl_3$) : δ 26,1 ($CH_2$), δ 29,4 ($CH_2$), δ 29,5 ($CH_2$), δ 29,6 ($CH_2$), δ 58,0 ($CH_2$), δ 70,3 ($CH_2$), δ

74,1 (CH), δ 80,1 (C).

12-propargyloxydodécan-1-ol

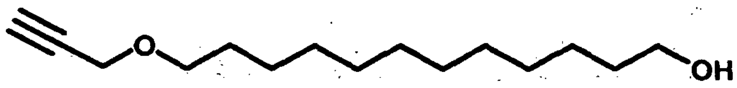

RF : 0,10 (Hexane/AcOtEt 9/1)
Rendement: 36 %
RMN $^{1}H$ (300,13 MHz, $CDCl_3$) : δ 1,32 (m, 16H, $CH_2$), δ 1,59 (m, 4H, $CH_2$), δ 2,43 (t, *j* = 3 Hz, 2H, CH), δ 3,52 (t, *j* = 6 Hz, 2H, $CH_2$), δ 3,65 (t, *j* = 6 Hz, 2H, $CH_2$), δ 4,15 (d, *j* = 3 Hz, 2H; $CH_2$).
RMN $^{13}C$ (75,47 MHz, $CDCl_3$) : δ 25,8 ($CH_2$), δ 26,0 ($CH_2$), δ 29,4 (2 $CH_2$), δ 29,5 (2 $CH_2$), δ 29,6 ($CH_2$), δ 32,7 ($CH_2$), δ 57,9 ($CH_2$), δ 62,7 ($CH_2$), δ 70,2 ($CH_2$), δ 74,2 (CH), δ 79,9 (C).

Préparation 7

*o*-propargylcholestérol

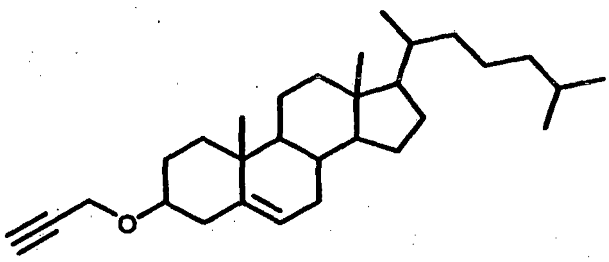

Dans un ballon propre et sec, sous atmosphère d'azote anhydre, on introduit 500 mg de cholestérol (1,3 mmol) en solution 0,5 M dans le DMF. La solution est alors refroidie à 0°C et 47 mg d'hydrure de sodium (1,5 équivalents, 2 mmol) sont additionnés par petites fractions. On laisse revenir le milieu réactionnel à température ambiante 238 mg de bromure de propargyle (1,5 équivalents, 2 mmol) sont ajoutés. L'agitation est maintenue pendant 5 h. La réaction est alors stoppée par ajout de 10 mL de méthanol et l'agitation est maintenue pendant 30 min. On ajoute au mélange 50 mL de $CH_2Cl_2$ puis on lave successivement par 2 fois 20 mL et 20 mL d'une solution saturée de NaCl. La phase organique est alors séchée sur $Na_2SO_4$ puis le solvant est éliminé sous pression réduite. Le composé attendu est obtenu après purification sur colonne chromatographique (Hexane/AcOEt 8/2). 215 mg d'un produit blanc sont ainsi isolés.
RF : 0,83 (Hexane/AcOEt 8/2)
Rendement : 39 %

Exemple 1

Thymidine 3'-(1,2-dimyristoyl-*sn*-glycéro-3-phosphate) (di c14dT)

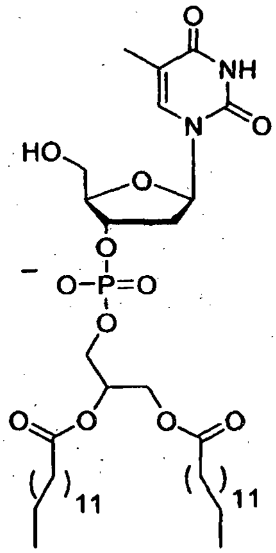

De la 5'-O-(4,4'-diméthoxytrityl)-2'-déoxythymidine,3'-[(2-cyano-éthyl)-N,N-diisopropyl)] phosphoramidite (0,500 g, 1 éq, 0,67 mmol), du 1,2-dimyristoyl-*sn*-glycérol (0,447 g, 1,3 éq, 0,87 mmol) et une solution de tétrazole 0,45 M dans l'acétonitrile (2 mL, 1,3 éq, 0,87 mmol) sont dissous dans 4 mL d'acétonitrile anhydre sous azote. Le milieu réactionnel est mis sous agitation magnétique pendant 24h00 à température ambiante. Le mélange est ensuite oxydé par ajout de 43 mL d'une solution de diiode 0,02M dans THF/Pyr/$H_2O$. Après 12 h à température ambiante, le solvant est évaporé sous vide. Le résidu est dissous dans 8 mL de dichlorométhane. Ensuite, 0,2 mL de 1,5-diazabicyclo[5.4.0] undec-7-ène (DBU) (1,3 éq, 0,87 mmol) sont ajoutés au milieu réactionnel pendant 5 h. Le milieu réactionnel est lavé avec une solution de HCl 0,1N puis avec une solution saturée de $Na_2S_2O_7$. La phase organique est concentrée sous vide. Le composé est obtenu après purification par chromatographie flash (381 mg) en utilisant un gradient d'élution (MeOH/DCM 9:1 jusqu'à 1:1).
Rendement: 69%
Rf: 0,34 (DCM/MeOH 9 :1)
*RMN 1H (300 MHz, CDCl3)* : δ en ppm 0,84 (t, 6H, J=6,92 Hz, 2*$CH_3$), 1,21 (m, 40H, 20*$CH_2$), 1,42 (dd, 4H, J1=8,45 Hz, J2=15,68 Hz, 2*CH2), 1,89 (s, 3H, Me), 2,30 (dd, 4H, J1=7,43 Hz, J2=15,92 Hz, 2*CH2), 2,83. (t, 2H, J=5,84, H2'), 3,84 (m, 1H, H3'), 4,09-4,35 (m, 7H, 2*$CH_2$(glycerol), H4', H5'), 5,27 (s, 1H, CHglycérol), 6,22 (t, 1H, J=6,81 Hz, H1'), 7.61 (s, 1H, Hbase).
*RMN 13C (75 MHz, CDCl3)* : δ en ppm 19,29 ($CH_3$), 23,71 ($CH_2$), 26,57 ($CH_2$), 28,73 ($CH_2$), 32,76 ($CH_2$), 37,85 ($CH_2$), 48,90 ($CH_2$), 166,15 (C=O).
*RMN 31P (121 MHz, CDCl3)* : δ en ppm 0,61.
Masse Haute Résolution FAB- théorique m/z = 815,4823 observé m/z = 815,4794.

Exemple 2

Thymidine 3'-(1,2-dipalmitoyl-*sn*-glycéro-3-phosphate) (di c16dT)

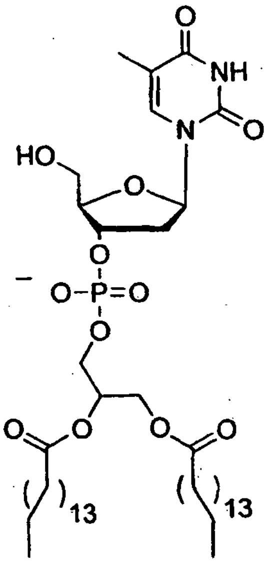

De la 5'-O-(4,4'-diméthoxytrityl)-2'-déoxythymidine,3'-[(2-cyano-éthyl)-N,N-diisopropyl)] phosphoramidite (0,500 g, 1 éq, 0,67 mmol), du 1,2-dipalmitoyl-*sn*-glycérol (0,496 g, 1,3 éq, 0,87 mmol / solubilisé dans 3 mL de THF) et une solution de tétrazole 0,45 M dans l'acétonitrile (2 mL, 1,3 éq, 0,87 mmol) sont dissous dans 3 mL d'acétonitrile anhydre sous azote. Le milieu réactionnel est mis sous agitation magnétique pendant 24h00 à température ambiante et sous azote. Le mélange est ensuite oxydé par ajout de 43 mL d'une solution de diiode 0,02M dans THF/Pyr/$H_2O$. Après 12 h à température ambiante, le solvant est évaporé sous vide et séché à la pompe sous $P_2O_5$ pendant une nuit. Le résidu est dissous dans 8 mL de dichlorométhane. Ensuite, 0,2 mL de 1,5-diazabicyclo[5.4.0]undec-7-ène (DBU) (1,3 éq, 0,87 mmol) sont ajoutés au milieu réactionnel pendant 5 h. Le milieu réactionnel est lavé avec une solution de HCl 0,1N puis avec une solution saturée de $Na_2S_2O_3$. La phase organique est concentrée sous vide. Le composé est obtenu après purification par chromatographie flash (180 mg) en utilisant un gradient d'élution (MeOH/DCM 98:2 jusqu'à 1:1).
Rendement : 24%
Rf : 0,3 (DCM/MeOH 8:2)
*RMN 1H (300 MHz, CDCl3)* : δ en ppm 0,88 (t, 6H, J=6,9 Hz, 2*$CH_3$), 1,25 (m, 48H, 24*$CH_2$), 1,42 (dd, 4H, J1=8,4 Hz, J2=15,6 Hz, 2*CH2), 1,90 (s, 3H, Me), 2,33 (m, 4H, 2*$CH_2$), 2,83 (t, 2H, J=5,6 Hz, $H_2$'), 3,84 (m, 1H, H3'), 4,09-4,35 (m, 7H, 2*$CH_2$(glycerol), $H_4$', $H_5$'), 5,27 (s, 1H, CH glycérol), 6,21 (t, 1H, J=6,7 Hz, H1'), 7,54 (s,1H, H base).
*RMN 13C (75 MHz, CDCl3)* : δ en ppm 12,4 ($CH_3$ base), 14,1 ($CH_3$ chaîne), 19,6 ($CH_2$), 19,7 ($CH_2$), 22,6 ($CH_2$), 24,8 ($CH_2$), 29,1-29,6 (CH2), 31,9 ($CH_2$), 33,9 ($CH_2$), 34,1 ($CH_2$), 61,5 ($CH_2$), 61,7 ($CH_2$), 62,5 ($CH_2$), 62,6 ($CH_2$), 66,1 ($CH_2$), 66,2 ($CH_2$), 69,1 (CH), 78,8 (CH), 85,5 (CH), 86,1 (CH), 111,3 (C base), 136,8 (CH base), 150,5 (C=O base), 164,1 (C=O base), 173,0 (C=O chaîne), 173,5 (C=O chaîne).
*RMN 31P (121 MHz, CDCl3)* : δ en ppm 2,1.
Masse ESI- : théorique m/z = 872,5 observé m/z = 871,3.

Exemple 3

5'-(4-Hexadécyloxyméthyl-[1,2,3]triazol-1-yl)-5',2'didéoxythymidine

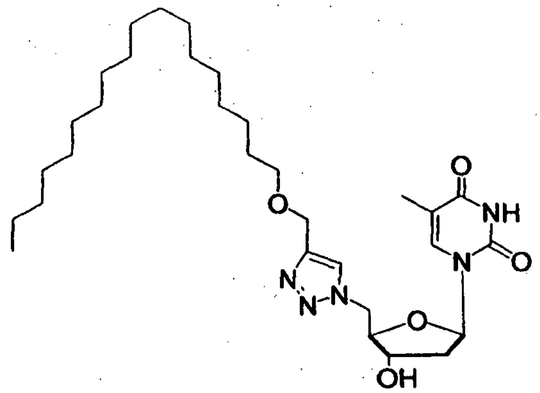

Dans un ballon, on introduit 200 mg de 5'-azido-5'-déoxythymidine telle que décrite dans la préparation 3 (0,75 mmol) et 231 mg de 1-propargyloxyoctadécane telle que décrit dans la préparation 5 (1 équivalent) en solution 0,1 M dans un mélange de THF et d'eau (1/1). On ajoute alors, successivement : 30 mg de d'ascorbate de sodium (0,2 équivalents, 0,15 mmol) et 12 mg de sulfate de cuivre (0,1 équivalent, 0,075 mmol). Le milieu réactionnel est agité et chauffé à 60°C pendant 5 h. Le mélange est alors refroidi à température ambiante. Le milieu réactionnel est immédiatement adsorbé sur silice et le solvant éliminé par évaporation. 180 mg d'un solide blanc sont obtenus après chromatographie sur colonne de silice (AcOEt/MeOH 8/2).
RF : 0,72 (AcOEt/MeOH 8/2)
Rendement : 42 %
SM Haute résolution $[M+H]^+$ : masse calculée : 576,4125 , masse mesurée : 576,4120

<u>Exemple 4</u>

5'-(4-Hexadécyloxyméthyl-[1,2,3]triazol-1-yl)-5',2'-didéoxyadénosine

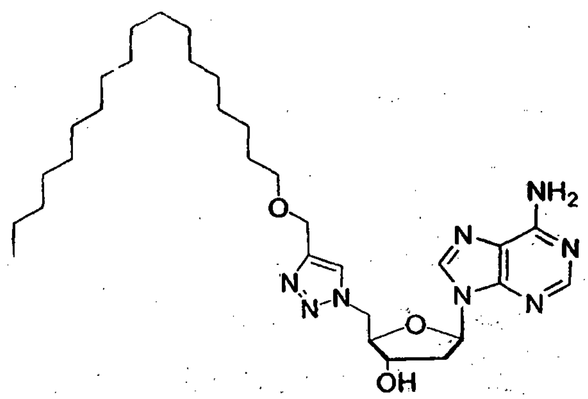

Dans un ballon, on introduit 200 mg de 5'-azido-5',2'-didéoxyadénosine telle que décrite dans la préparation 4 (0,72 mmol) et 223 mg de 1-propargyloxyoctadécane telle que décrite dans la préparation 5 (1 équivalent) en solution 0,1 M dans un mélange de THF et d'eau (1/1). On ajoute alors, successivement : 30 mg de d'ascorbate de sodium (0,2 équivalents, 0,15 mmol) et 12 mg de sulfate de cuivre (0,1 équivalent, 0,075 mmol). Le milieu réactionnel est agité et chauffé à 60°C pendant 5 h, puis le mélange est refroidi à température ambiante. Le milieu réactionnel est immédiatement adsorbé sur silice et le solvant éliminé par évaporation. 150 mg d'un solide blanc sont obtenus après chromatographie sur colonne (AcOEt/MeOH 8/2).
RF : 0,65 (AcOEt/MeOH 8/2)
Rendement: 35 %
RMN $^1H$ (300,13 MHz, $CDCl_3$) : $\delta$ 0,89 (t, *j* = 6 Hz, 3H, $CH_3$), $\delta$ 1,26 (m, 30H, $CH_2$), $\delta$ 1,55 (m, 2H, $CH_2$), $\delta$ 2,54 (m, 1H, $CH_2$), $\delta$ 3,06 (m, 1H, $CH_2$), $\delta$ 3,45 (t, *j* = 6 Hz, 2H, $CH_2$), $\delta$ 4,50 (m, 4H, $CH_2$, CH), $\delta$ 4,89 (m, ???), $\delta$ 5,88 (s, 2H, $NH_2$), $\delta$ 6,40 (t, *j* = 6 Hz, 1H, CH), $\delta$ 7,42 (s, 1H, CH), $\delta$ 7,81 (s, 1H, CH), $\delta$ 8,35 (s, 1H, CH).
SM Haute résolution $[M+H]^+$ : massé calculée : 585,4241 , masse mesurée: 585,4254

Exemple 5

5'-(4-((O-cholestéryl)-méthyl)-[1,2,3]triazol-1-yl)-5',2'didéoxythymidine

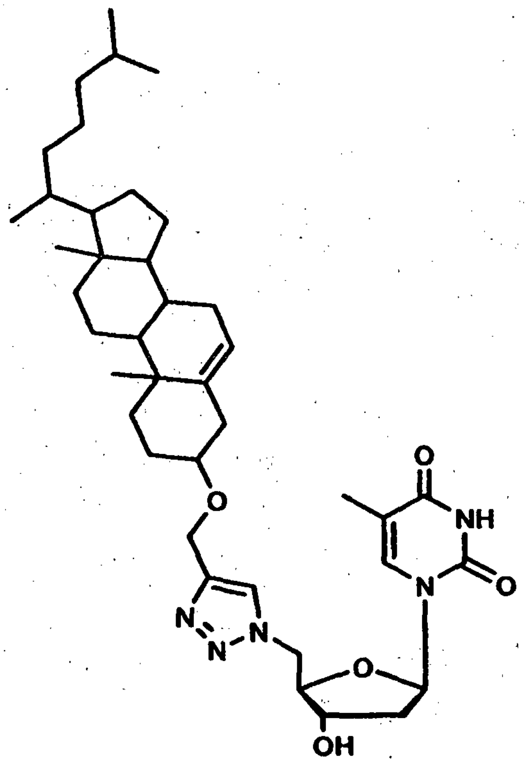

Dans un ballon, on introduit 170 mg de 5'-azido-5'-déoxythymidine telle que décrite dans la préparation 3 (0,63 mmol) et 270 mg de *o*-propargylcholestérol telle que décrit dans la préparation 7 (1 équivalent) en solution 0,1 M dans un mélange THF eau (1/1). On ajoute, successivement : 20 mg de d'ascorbate de sodium (0,2 équivalent, 0,13 mmol) et 10 mg de sulfate de cuivre (0,1 équivalent, 0,063 mmol). Le milieu réactionnel est agité et chauffé à 60°C pendant 5 h. Le mélange est refroidi à température ambiante. Le milieu réactionnel est immédiatement adsorbé sur silice et le solvant éliminé par évaporation. Le composé est obtenu pur par chromatographie sur colonne (AcOEt/MeOH 8/2). 260 mg d'un solide blanc sont obtenus.
RF : 0,57 (AcOEt/MeOH 8/2)
Rendement : 59 %
SM $[M+H]^+$ : 692,3

Exemple 6

1,12-bis-[5'-(4-(méthyl)-[1,2,3]triazol-1-yl)-5',2'didéoxythymidine]-oxydodécane

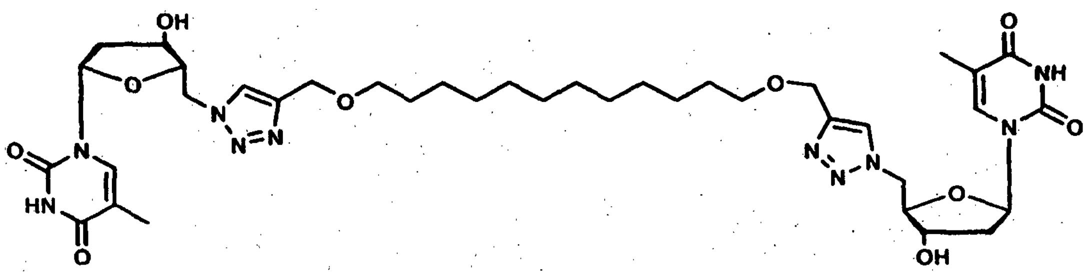

Dans un ballon, on introduit 100 mg de 5'-azido-5'-déoxythymidine telle que décrite dans la préparation 3 (0,375 mmol) et 52 mg de 1,12-dipropargyloxydodécane préparé à partir du composé décrit dans la préparation 6 (0,5 équivalent) en solution 0,1 M dans un mélange THF eau (1/1). On ajoute, successivement : 15 mg de d'ascorbate de sodium (0,2 équivalent, 0,075 mmol) et 6 mg de sulfate de cuivre (0,1 équivalent, 0,0375 mmol). Le milieu réactionnel est agité et chauffé à 60°C pendant 5 h. Le mélange est alors refroidi à température ambiante. Le milieu réactionnel est immédiatement adsorbé sur silice et le solvant éliminé par évaporation. Le composé est obtenu pur par chromatographie sur colonne (AcOEt/MeOH 8/2). 90 mg d'un solide blanc sont obtenus.
Rendement : 59 %
RMN $^1H$ (300,13 MHz, MeOH $d_4$): δ 1,28 (m, 16H, $CH_2$), δ 0,83 (m, 4H, $CH_2$), δ 1,89 (s, 6H, $CH_3$), δ 2,17 (s, 2H, $CH_2$), δ 2,25 (m, 4H, $CH_2$), δ 3,51 (t, *j* = 6 Hz, 4H, $CH_2$), δ 4,18 (m, 2H, CH) δ 4,42 (m, 2H, OH) δ 4,58 (s, 4H, $CH_2$), δ 4,76 (qd, *j* = 6 Hz, 4H, $CH_2$), δ 6,21 (t, *j* = 6 Hz, 2H, CH), δ 7,23 (s, 2H, CH), δ 7,99 (s, 2H, CH).

Exemple 7

5'-(4-(1 (*R*)-hydroxy-hexyl)-[1,2,3]triazol-1-yl)-5',2'didéoxythymidine

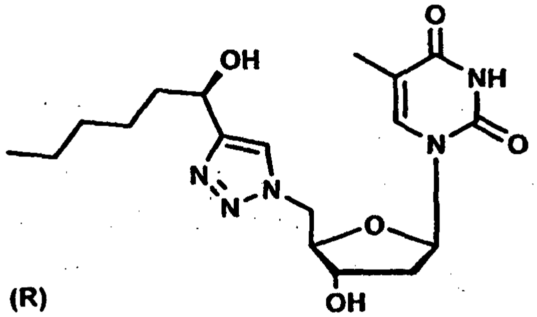

(R)

Dans un ballon, on introduit 215 mg de 5'-azido-5'-déoxythymidine telle que décrite dans la préparation 3 (0,8 mmol) et 101,5 mg de (R) oct-1-yn-3-ol (1 équivalent) en solution 0,1 M dans un mélange THF eau (1/1). On ajoute, successivement : 31,5 mg de d'ascorbate de sodium (0,2 équivalent, 0,15 mmol) et 13 mg de sulfate de cuivre (0,1 équivalent, 0,075 mmol). Le milieu réactionnel est agité et chauffé à 60°C pendant 5 h. Le mélange est alors refroidi à température ambiante. Le milieu réactionnel est alors immédiatement adsorbé sur silice et le solvant éliminé par évaporation. Le composé est obtenu pur par chromatographie sur colonne (AcEt/MeOH 9/1). 240 mg d'un solide blanc sont obtenus.
RF : 0,48 (AcEt/MeOH 9/1)
Rendement : 76 %
SM Haute résolution $[M+H]^+$: masse calculée : 576,4125 , masse mesurée: 576,4120

Exemple 8

5'-(4-(1(*S*)-hydroxy-hexyl)-[1,2,3]triazol-1-yl)-5',2'didéoxythymidine

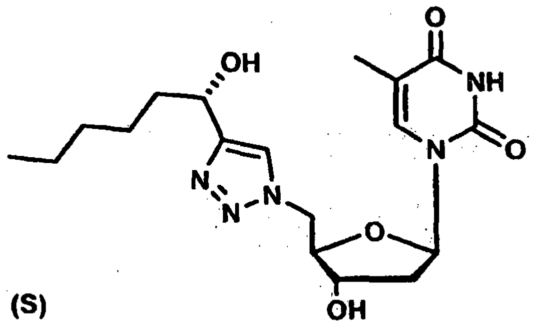

(S)

Dans un ballon, on introduit 215 mg de 5'-azido-5'-déoxythymidine telle que décrite dans la préparation 3 (0,8 mmol) et 101,5 mg de (S) oct-1-yn-3-ol (1 équivalent) en solution 0,1 M dans un mélange THF eau (1/1). On ajoute, successivement : 31,5 mg de d'ascorbate de sodium (0,2 équivalent, 0,15 mmol) et 13 mg de sulfate de cuivre (0,1 équivalent, 0,075 mmol). Le milieu réactionnel est agité et chauffé à 60°C pendant 5 h. Le mélange est alors refroidi à température ambiante. Le milieu réactionnel est alors immédiatement adsorbé sur silice et le solvant éliminé par évaporation. Le composé est obtenu pur par chromatographie sur colonne (AcOEt/MeOH 85/15). 255 mg d'un solide blanc sont obtenus.
RF : 0,48 (AcOEt/MeOH 85/15)
Rendement: 78 %
SM Haute résolution $[M+H]^+$ : masse calculée : 576,4125 , masse mesurée : 576,4120

Exemple 9

5'-(4-(1-hydroxy-hexyl)-[1,2,3]triazol-1-yl)-5',2'didéoxyadenosine

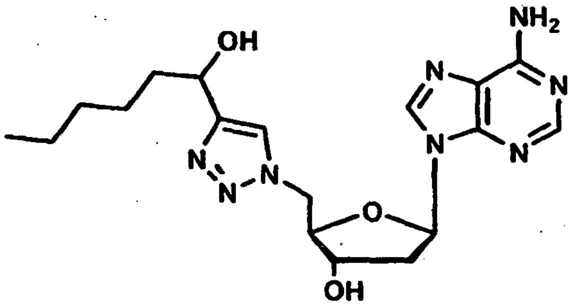

Dans un ballon, on introduit 200 mg de 5'-azido-5'-déoxythymidine telle que décrite dans la préparation 3 (0,75 mmol) et 95 mg du mélange racémique d'oct-1-yn-3-ol (1 équivalent) en solution 0,1 M dans un mélange THF eau (1/1). On ajoute, successivement : 30 mg de d'ascorbate de sodium (0,2 équivalent, 0,15 mmol) et 12 mg de sulfate de cuivre (0,1 équivalent, 0,075 mmol). Le milieu réactionnel est agité et chauffé à 60°C pendant 5 h. Le mélange est alors refroidi à température ambiante. Le milieu réactionnel est immédiatement adsorbé sur silice et le solvant éliminé par évaporation. Le composé est obtenu pur par chromatographie sur colonne (AcOEt/MeOH 8/2). 240 mg d'un solide blanc sont obtenus.
RF : 0,47 (AcOEt/MeOH 8/2)
Rendement : 80 %
SM Haute résolution $[M+H]^+$: masse calculée : 576,4125 , masse mesurée: 576,4120

Exemple 10: préparation des nanoparticules à compartiments multiples

On utilise comme composé de formule (I) anionique le composé thymidine 3'-(1,2-dipalmitoyl-sn-glycéro-3-phosphate) (di C16 dT) préparé à l'exemple 2 , comme co-lipide la dioléylphosphatidylcholine (DOPC) et comme composés de formule (I) cationique le composé(N-[5'-(2',3'-dioleoyl)uridine]-N',N',N'-trimethylammonium tosylate) (DOTAU) préparé comme décrit dans Pauline Chabaud et al., Bioconjugate Chem., 2006, 17, 466-472.

*1) Préparation des solutions stocks*

- a) Préparation de la solution de cis-platine :

  15 mg de cis-platine sont solubilisés dans 10 mL d'eau milliQ (concentration finale :5 mM). Cette suspension est agitée pendant 1 min (vortex), puis incubée à 37°C pendant 24 h.

- b) Préparation des solutions de lipides :

  Solution A : 20 mg du diC16dT sont solubilisés dans 2 mL de dichlorométhane (10 mg/mL). Cet échantillon est stocké à -20°C.

Solution B : DOPC : solution à 20 mg/mL dans le dichlorométhane stockée à -20°C.
Solution C : DOTAU : solution à 20 mg/mL dans le dichlorométhane stockée à -20°C.

2) Préparation de la formulations lipidique pour la première couche

Dans un tube eppendorf® de 2 mL, 52,3 μL de la solution A sont mélangés à 47,2 μL de la solution B. Ces volumes correspondent à un rapport de 1/1 des deux lipides en mole.

Le dichlorométhane est évaporé sous l'azote de manière à obtenir un film lipidique homogène.

3) Préparation des nanoparticules (première couche lipidique anionique)

1,2 mL de la solution de cis-platine pré-incubée à 37°C sont utilisés pour réhydrater le film lipidique préalablement préparé. Le mélange est incubé à température ambiante pendant une nuit. Une série de 10 cycles de chauffage (bain marie à 55°C) et de congélation (carboglace/méthanol -72°C) est réalisée.

4) Lavage et récupération des nanoparticules comportant une première couche lipidique anionique

Une fois la série de 10 cycles terminée, la suspension est agitée et mise dans un tube à hémolyse en verre puis soumise à une sonification pendant 7 min. Après la sonification, la suspension est centrifugée à 10 000 rpm/5min/20°C. Le surnagent est jeté et le culot des nanoparticules est resuspendu dans 1 mL d'eau milliQ. Cette étape est renouvelée une seconde fois.

La suspension est centrifugée à 1000 rmp/2,5min/20°C. Le culot est jeté et le surnageant contient les nanoparticules anioniques.

Le potentiel zeta, mesuré selon la technique décrite dans Andrea Mayer et al. Toxicology, 2009, 258, 139-147 ou K. Furusawa et K. Uchiyama, 1988, 140, 217-226, est de -43,3 ± 6 mV.

5) Préparation des nanoparticules comportant une première couche lipidique anionique et une deuxième couche lipidique cationique

Dans un tube eppendorf® de 2 mL, on dépose 180 μL de la solution C. Le dichlorométhane est évaporé avec de l'azote comprimé de manière à obtenir un film lipidique homogène.

6) Lavage et récupération des nanoparticules à compartiments multiples (couche de surface cationique)

Le film lipidique cationique est réhydraté avec la suspension des nanoparticules anioniques préparée à l'étape 4).

Une agitation au vortex pendant 5 min est réalisée suivie d'une sonification d'une minute.

La suspension est centrifugée à 10,000 rpm pendant 5 min à 20 °C afin d'éliminer les lipides non liés aux nanoparticules. le surnageant est éliminé puis le culot est réhydraté avec 1 mL d'eau milliQ.

La suspension est centrifugée à 1000 rmp/2,5min/20°C. Le culot est jeté et le surnageant contient les nanoparticules multi-compartiments à couche de surface cationique.

Le potentiel zeta, mesuré comme précedemment, est de 42,3 ± 8mV.

Exemple 11 : test de stabilité (% de Cis-platine libéré)

Les nanoparticules préparées selon le protocole de l'exemple 10 sont dosées en ICP-optique (la valeur mesurée correspond à la concentration totale). La suspension des nanoparticules est aliquotée dans 5 tubes eppendorf® (150 μL). Ces derniers sont incubés à 37°C sous agitation (300 rpm) pendant différents temps (0, 2,5, 5, 10 et 24 h).

A un temps donné (x), le tube est centrifugé à 14000 rpm/10min/20°C et 50 μL de surnageant (récupéré doucement pour ne pas remettre le culot en suspension) sont dosés.

Des nanoparticules à base de1,2-dioléoyl-sn-glycéro-3-[phospho-L-sérine] (DOPS) avec de la 1,2-dioléoyl-sn-glyéro-3-phosphocholine (DOPC) comme colipide sont préparées selon le même protocole à titre de comparaison. Ces nanoparticules comprennent une seule couche lipidique anionique.

Le pourcentage de libération de cis-platine est calculé selon l'équation suivante :

$$\text{\% de cis-platine libéré} = Cx\text{-}C0/Ct\text{-}C0$$

Cx : concentration trouvée à un temps donné (x).
C0 : concentration trouvée dans le surnageant avant incubation.
Ct : concentration totale trouvé sans incubation et sans centrifugation.

La courbe de libération du cis-platine en fonction du temps d'incubation représentés sur la figure 1.

Les nanoparticules comportant une seule couche lipidique anionique obtenues à l'issue de l'étape 4 de l'exemple 10 (notées NP-) sont représentées par le symbole -♦-, les nanoparticules selon l'invention (notées NP+) comportant une première couche lipidique anionique et une deuxième couche lipidique cationique obtenues à l'issue de l'étape 6 de l'exemple 10 sont représentées par le symbole **-■-** et les nanoparticules à base de DOPC/DOPS (notées PS) par le symbole -▲-

Les résultats montrent que le temps de demi-vie (temps d'incubation nécessaire pour libérer 50% du cis-platine) est supérieur à 24 h pour les nanoparticules NP+ selon l'invention, alors qu'il est de l'ordre de 6,5 h pour les nanoparticules à base de DOPC/DOPS.

En outre, après le même temps d'incubation (6,5h), on observe que les nanoparticules NP- (monocouche) ont libéré plus de 30% de leur contenu en cis-platine, alors que les nanoparticules NP+ selon l'invention ont libéré moins de 20% de cis-platine, ce qui souligne les propriétés remarquables de stabilité des nanoparticules NP+ à 37°C.

Exemple 12 : test de stabilité (% de Cis-platine libéré) en présence de sérum de veau foetal

Les nanoparticules préparées selon le protocole de l'exemple 10 sont dosées en ICP-optique (la valeur mesurée correspond à la concentration totale). La suspension des nanoparticules est aliquotée dans 5 tubes eppendorf® (150 μL). Ces derniers sont centrifugés à 10,000 rpm pendant 5 min à 20°C. 50 μL du surnagent est dosé par ICP optique et les 100 μL restant sont écartés. Le culot contenant les nanoparticules est réhydraté avec 150 μL de sérume de veau foetal (SVF, ref invitrogen10270-106). Les échantillons sont incubés à 37°C sous agitation (300 rpm) pendant différents temps (0, 2,5, 5, 10 et 24 h).

A un temps donné (x), le tube est centrifugé à 14000 rpm/10min/20°C et 50 μL de surnageant (récupéré doucement pour ne pas remettre le culot en suspension) sont dosés.

Des nanoparticules à base de1,2-dioléoyl-sn-glycéro-3-[phospho-L-sérine] (DOPS) avec de la 1,2-dioléoyl-sn-glyéro-3-phosphocholine (DOPC) comme colipide sont préparées selon le même protocole à titre de comparaison.

Le pourcentage de libération de cis-platine est calculé selon l'équation suivante :

$$\% \text{ de cis-platine libéré} = Cx\text{-}C0/Ct\text{-}C0$$

Cx : concentration trouvée à un temps donné (x).
C0 : concentration trouvée dans le surnageant avant incubation et avant contact avec le SVF.
Ct : concentration totale trouvé sans incubation et sans centrifugation.

La courbe de libération du cis-platine en fonction du temps d'incubation est représentée sur la figure 2.

Les nanoparticules selon l'invention (notées NP+) comportant une première couche lipidique anionique et une deuxième couche lipidique cationique obtenues dans l'exemple 10 sont représentées par le symbole -■-et les nanoparticules à base de DOPC/DOPS (notées PS) par le symbole -▲-

Les résultats montrent que le temps d'incubation nécessaire pour libérer 90% du cis-platine est supérieur à 24 h pour les nanoparticules NP+ selon l'invention, alors qu'il est inferieur à 2h pour les nanoparticules PS.

Exemple 13 : dosage du cis-platine intracellulaire

Protocole

Des cellules IGROV1 (lignée d'adénocarcinome ovarien sensibles au cis platine) à 80 % de confluence (boîte de 10 cm de diamètre) sont traitées avec 100 μM de cis-platine libre ou encapsulé dans les nanoparticules de l'exemple 10 pendant 2, 4 ou 6 h. Au terme de ce traitement deux lavages au PBS sont effectués. Les cellules sont traitées avec de la trypsine et remises en suspension dans du PBS. Deux lavages au PBS des suspensions cellulaires sont effectués (centrifugation 1000 rpm/1min). Les cellules sont mises en suspension dans 1 mL de PBS et comptées.

On procède de la même manière avec des cellules SKV03 (lignée d'adénocarcinome ovarien résistantes au cis-platine).

Dosage en ICP-optique

$10^6$ cellules sont lysées avec 500 μL de la solution de lyse cellulaire (lysis buffer de chez SIGMA). Le volume est complété avec de l'eau milliQ à 1% d'acide $HNO_3$ pour atteindre 5 mL.

Résultats

Les résultats sont représentés sur la figure 3, qui montre la concentration en cis-platine libéré après lyse cellulaire (exprimée en nanomole/$10^6$ cellules/100μM de traitement) en fonction du temps, correspondant à la concentration en cis-platine internalisé dans les cellules traitées.

Les colonnes hachurées verticalement (à gauche), horizontalement (centre) et pointillées (à droite) correspondent à un traitement des cellules pendant 2h, 4h et 6h respectivement.

Les résultats montrent que l'internalisation de cis-platine est nettement plus efficace en présence des nanoparticules selon l'invention (notées NP+) comportant une première couche lipidique anionique et une deuxième couche lipidique cationique obtenues dans l'exemple 10 que dans le cas des nanoparticules PS et du cis-platine libre.

Par exemple, dans des conditions identiques ($10^6$ cellules IGROV1, 100 μM, 2 h) 0,5 nanomole de cis-platine est internalisé dans le cas du cis-platine libre alors que l'internalisation est 40 fois plus importante (20 nanomoles) dans le cas des nanoparticules NP+ selon l'invention (figure 3A).

En ce qui concerne la lignée cellulaire résistante au cisplatine SKOV3, dans des conditions identiques ($10^6$ cellules SKOV3, 1.00 μM, 2 h), l'internalisation est 60 fois plus importante (30 nanomoles) dans le cas des nanoparticules NP+ selon l'invention que pour le cis-platine libre (0,5 nanomole) (figure 3B).

Exemple 14 : Etude des effets cytotoxiques des nanoparticules à compartiments multiples sur différentes lignées tumorales

Protocole

*a/ Préparation et traitement des cellules :*

L'étude consiste à déterminer la concentration inhibant 50% de prolifération cellulaire 50 ($CI_{50}$) sur un panel de lignées tumorales, à savoir

- A2780 (Human ovarian carcinoma epithelial tumor) sensible au cis-platine et A2780/CisPt résistante au cis-platine (Human ovarian carcinoma epithelial tumor)
- IGROV-1 sensible (Human ovarian carcinoma) au cis-platine et IGROV-1/CisPt résistante au cis-platine (Human ovarian carcinoma)
- L1210 (mouse lymphocytic leukemia) sensible au cis-platine et L1210/CisPt (mouse lymphocytic leukemia) résistante au cis-platine (leucémie humaine)
- NIH : OVCAR3 (carcinome ovarien), et
- P388 (mouse lymphoma)

Dans une plaque 96 puits, 2500 cellules (lignées d'adénocarcinomes ovariens, etc) par puits sont incubées dans 100 μL du milieu avec sérum. Après 24 h le milieu est aspiré et les cellules sont traitées avec du cis-platine libre ou encapsulé dans les nanoparticules de l'exemple 10 dans 100 μL du milieu sans sérum à différentes concentrations (500, 100, 10, 1, 0.1, 0.01, 0.001 μM). Après 24 h de traitement, le milieu est retiré et les cellules sont lavées 2 fois avec 100 μL de PBS puis incubées avec 100 μL du milieu avec sérum.

*b/ Révélation de la toxicité :*

48h après les deux lavages, la viabilité cellulaire est révélée en ajoutant 20 μL de MTS. L'absorbance à 490 nm est mesurée après 2 à 4 h d'incubation à 37°C. L'absorbance est proportionnelle à la viabilité cellulaire.

*c/ résultats*

Les résultats sont représentés sur la figure 4, qui montre la concentration nécessaire pour obtenir 50 % de mort cellulaire (IC50) avec du cis-platine libre (colonne gris clair à gauche) ou les nanoparticules selon l'invention comportant une première couche lipidique anionique et une deuxième couche lipidique cationique obtenues à l'issue de l'étape 6 de l'exemple 10 (notées NP+) contenant du cis-platine (colonne gris foncé à droite).

Les résultats montrent que les nanoparticules selon l'invention sont plus efficaces que le cis-platine libre dans toutes les lignées cellulaires étudiées sensibles ou résistantes au cis-platine.

Par exemple, pour la lignée cellulaire A2780 sensible au cis-platine, on obtient 50 % de mort cellulaire avec 0,23 μM de nanoparticules selon l'invention alors qu'il faut utiliser 4,68 μM de cis-platine libre pour obtenir la même mortalité.

Sur la lignée A2780 résistante au cis-platine; on obtient 50 % de mort cellulaire avec 0,21 μM de nanoparticules selon l'invention contre 29,21 μM de cis-platine libre. Dans ce cas les nanoparticules selon l'invention sont respectivement 18 et 140 fois plus efficaces que le cis-platine libre sur les lignées A2780 sensibles et A2780 résistantes.

Exemple 15 : Etude des effets cytotoxiques des nanoparticules à compartiments multiples sur les lignées tumorales IGROV-1 et SKOV3

Protocole

*a/Prépararion et traitement des cellules :*

Dans une plaque 96 puits, 2500 cellules (IGROV1, SKOV3, lignées d'adénocarcinomes ovariens) par puits sont incubées dans 100 μL du milieu avec sérum. Après 24 h le milieu est aspiré et les cellules sont traitées avec du cis-platine libre ou encapsulé dans les nanoparticules de l'exemple 10 dans 100 μL du milieu sans sérum à différentes concentrations (500, 100, 10, 1, 0.1, 0.01, 0.001 μM). Après 24 heures de traitement, le milieu est retiré et les cellules sont lavées 2 fois avec 100 μL de PBS puis incubées avec 100 μL du milieu avec sérum.

*b/ Révélation de la toxicité :*

48 h après les deux lavages, la viabilité cellulaire est révélée en ajoutant 20 μL de MTS. L'absorbance à 490 nm est mesurée après 2 à 4 heures d'incubation à 37°C. L'absorbance est proportionnelle à la viabilité cellulaire.

*c/ résultats*

Les résultats sont représentés sur la figure 5, qui montre la concentration nécessaire pour obtenir 50 % de mort cellulaire (IC50) avec du cis-platine libre (colonne 1, gauche), les nanoparticules contrôle à base de DOPC/DOPS (notées PS) (colonne 2, centre gauche), les nanoparticules comportant une seule couche lipidique anionique obtenues à l'issue de l'étape 4 de l'exemple 10 (notées NP-) (colonne 3, centre droite) et les nanoparticules selon l'invention (notées NP+) comportant une première couche lipidique anionique et une deuxième couche lipidique cationique obtenues à l'issue de l'étape 6 de l'exemple 10 (colonne droite).

La figure 5A concerne la lignée cellulaire IGROV1 et la figure 5B concerne la lignée cellulaire SKOV3.

Les résultats montrent les nanoparticules NP+ contenant du cis-platine selon l'invention sont plus efficaces que le cis-platine libre dans les deux lignées cellulaires, IGROV1 (sensible au cis-platine) et SKOV3 (résistante au cis-platine).

Sur la lignée IGROV1, on obtient 50 % de mort cellulaire avec 0,18 μM de nanoparticules contenant du cis-platine NP+ alors qu'il faut utiliser 2,41 μM de cis-platine libre pour obtenir ce résultat. Sur la lignée SKOV3, on obtient 50 % de mort cellulaire avec 0,29 μM de nanoparticules NP+ contenant du cis-platine contre 4,3 μM de cis-platine libre.

Les nanoparticules contenant du cis-platine selon l'invention (NP+) sont respectivement 13 et 14 fois plus efficaces que le cis-platine libre sur les lignées IGROV1 et SKOV3 respectivement.

Exemple 16 : mise en évidence de la structure à compartiments multiples des nanoparticules selon l'invention

Des formulations de nanoparticules ont été préparées avec différents marqueurs, afin d'étudier, d'une part, la localisation du marqueur dans la couche lipidique, et, d'autre part, l'état des nanoparticules après leur entrée dans les cellules.

Des sondes fluorescentes lipophiles ont été insérées dans les formulations en tant que marqueur, d'une part, et en tant que composé lipidique mimant une pro-drogue (par exemple, un conjugué lipidique analogue d'un nucléoside anticancéreux tel que le 5-FU) d'autre part.

Les différentes formulations suivantes ont été réalisées :

Formulation A : diC16dT/DOPC 50/50
Formulation B : DOTAU/DOPC 50/50
Formulation C : diC16dT/DOPC/DOPE/fluorescéine 49,25/49,25/0,5 (λ ex = 483 nm, (λ ém = 518 nm)

Formulation D : DOTAU/DOPC/DOPE/rhodamine 49,25/49,25/0,5 ($\lambda$ ex = 550 nm, ($\lambda$ ém = 590 nm)

On a ainsi préparé les nanoparticules NP1, NP2 et NP3 selon l'invention de différentes compositions, définies comme suit :

| Nanoparticule | Première couche | Seconde couche |
|---|---|---|
| NP1 | formulation C | formulation B |
| NP2 | formulation A | formulation D |
| NP3 | formulation C | formulation D |

Les nanoparticules NP1, NP2 et NP3 sont représentées sur la figure 6. Dans chacune des nanoparticules, la couche blanche représente une couche lipidique non marquée, la couche grise représente la couche marquée par la fluorescéine (formulation C), la couche noire représente la couche marquée par la rhodamine (formulation D) et le centre en pointillés représente le cis-platine incorporé.

Des mesures de FACS (Fluorescence Activated Cell Sorting) ont été réalisées sur des cellules SKV03 ayant été incubées :

- en absence de nanoparticules selon l'invention à titre de contrôle (figure 7A)
- en présence de nanoparticules NP1 dans lesquelles la première couche est marquée (figure 7B),
- en présence de nanoparticules NP2 dans lesquelles la deuxième couche est marquée (figure 7C), et
- en présence de nanoparticules NP3 dans lesquelles les deux couches sont marquées (figure 7D).

Les données de FACS obtenues montrent la présence des deux marqueurs fluorescents (fluorescéine, rhodamine) dans les cellules SKV03 après incubation en présence des nanoparticules NP3 portant ces marqueurs. Ces résultats montrent, d'une part, que les nanoparticules selon l'invention ont une structure à compartiment multiples, et, d'autre part, restent intactes après internalisation dans les cellules.

Des expériences de microscopie de fluorescence ont confirmé les résultats obtenus par FACS. Les images montrent que les nanoparticules marquées NP1, NP2 et NP3 sont internalisées intactes dans les cellules SKVO3.

**Revendications**

**1.** Formulation à compartiments multiples sous forme de nanoparticule ayant un diamètre moyen d'environ 1 à 200 nm constituée d'un coeur solide contenant un agent thérapeutique, entouré par au moins deux couches lipidiques de polarité différente formées à partir de molécules ou macromolécules amphiphiles fonctionnelles, dans laquelle chaque couche lipidique est constituée d'au moins un composé amphiphile fonctionnel de formule (I)

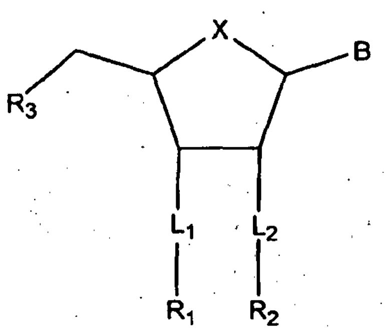

(I)

dans laquelle

- X représenté un atome d'oxygène, de soufre ou un groupe méthylène,
- B représente une base purique ou pyrimidique telle que uracile, adénine, guanine, cytosine, thymine, hypoxanthine, ou leurs dérivés, ou encore une base hétérocylique mono-ou bi-cyclique non naturelle dont chaque cycle

comporte 4 à 7 chaînons, éventuellement substituée;

- $L_1$ et $L_2$, identiques ou différents, représentent l'hydrogène, un groupe oxycarbonyl -O-C(O)-, un groupe thiocarbamate -O-C(S)-NH-, un groupe carbonate -O-C(O)-O-, un groupe carbamate -O-C(O)-NH-, un atome d'oxygène, un groupe phosphate, un groupe phosphonate ou un groupe hétéroaryle comprenant 1 à 4 atomes d'azote, substitué ou non substitué par une chaîne hydrocarbonée, saturée ou insaturée, linéaire ou ramifiée en $C_2$-$C_{30}$,

ou encore, $L_1$ et $L_2$, ensemble, forment un groupement cétal de formule

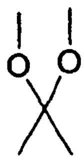

ou encore $L_1$ ou $L_2$ représente l'hydrogène, et l'autre représente un groupe hydroxy ou un groupe hétéroaryle comprenant 1 à 4 atomes d'azote, non substitué ou substitué par une chaîne alkyle linéaire ou ramifiée en $C_2$-$C_{30}$.

- $R_1$ et $R_2$, identiques ou différents, représentent

  - une chaîne hydrocarbonée linéaire ou ramifiée en $C_2$-$C_{30}$, de préférence en $C_6$-$C_{25}$, notamment en $C_8$-$C_{25}$, saturée ou partiellement insaturée, éventuellement totalement ou partiellement fluorée, non substituée ou substituée sur le carbone d'extrémité de chaîne par un atome de fluor ou par un ester ou un éther benzylique ou naphtylique, ou
  - une chaîne diacyle dans laquelle chaque chaîne acyle en $C_2$-$C_{30}$, ou
  - un groupe diacylglycérol, sphingosine ou céramide, ou
  - lorsque $L_1$ ou $L_2$ représente l'hydrogène, et l'autre représente un groupe hydroxy ou un groupe hétéroaryle comprenant 1 à 4 atomes d'azote, $R_1$ et $R_2$ n'existent pas ;

- $R_3$ représente

  - un groupement hydroxy, amino, phosphate, phosphonate, phosphatidylcholine, O-alkyl phosphatidylcholine, thiophosphate, phosphonium, $NH_2$-$R_4$, $NHR_4R_5$ ou $NR_4R_5R_6$ dans lesquels $R_4$, $R_5$ et $R_6$, identiques ou différents, représentent un atome d'hydrogène ou une chaîne alkyle linéaire ou ramifiée en $C_1$-$C_5$ ou hydroxyalkyle linéaire ou ramifié en $C_1$-$C_5$, ou
  - une chaîne alkyle linéaire ou ramifiée en $C_2$-$C_{30}$ éventuellement substituée par un groupe hydroxy , ou
  - un reste cyclodextrine, ou
  - un reste

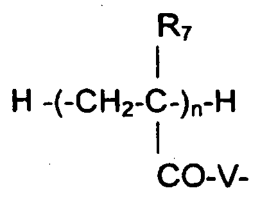

  dans lequel V représente une liaison -O-,-S-, ou -NH-, $R_7$ représente H ou $CH_3$, et n= 1 à 500, ou un groupe $-(CH_2)_n$-V-$R_8$, dans lequel $R_8$ représente un alkyle en $C_2$-$C_{30}$, et n = 1 à 500, ou
  - un groupe hétéroaryle renfermant 1 à 4 atomes d'azote, non substitué ou substitué par un alkyle en $C_2$-$C_{30}$, ou par un groupe $(CH_2)_m$-O-$(CH_2)_p$-$R_9$ dans lequel m = 1 à 6 et p = 0 à 10 et $R_9$ représente un groupe cétal cyclique renfermant 5 à 7 atomes de carbone, non substitué ou substitué par au moins un alkyle linéaire ou ramifié en $C_2$-$C_{30}$ ou par un reste stérol, ou encore

    - $R_3$ est lié par liaison covalente à un autre substituant $R_3$, identique ou différent, d'un autre composé de formule (I), identique ou différent, pour former un composé de formule (I) sous forme de dimère,

et chaque couche lipidique a une polarité différente de celle de la précédente.

**2.** Formulation selon la revendication 1, **caractérisée en ce que** chaque couche lipidique constitue un compartiment susceptible de comporter un agent thérapeutique identique à ou différent de celui présent dans le coeur.

**3.** Formulation selon l'une des revendications 1 ou 2, **caractérisée en ce qu'**un colipide peut être présent dans au moins une couche lipidique.

**4.** Formulation selon l'une quelconque des revendications 1 à 3, **caractérisée en ce que** l'agent thérapeutique est un agent anti-tumoral.

**5.** Formulation selon la revendication 4, **caractérisée en ce que** l'agent thérapeutique est choisi parmi les complexes de platine ou le ruthénium capable de se lier à des complexes de platines, ou encore les complexes inorganiques sans platine à base de ruthénium II ou III, de titane, de gallium, de cobalt, de fer ou d'or.

**6.** Formulation selon l'une quelconque des revendications 1 à 5, **caractérisée en ce que** l'agent thérapeutique est choisi parmi le cis-platine, le carboplatine, l'oxaliplatine, le nédaplatine et le lobaplatine.

**7.** Formulation selon l'une quelconque des revendications 1 à 6, **caractérisée en ce que**, dans la formule (I), X représente l'oxygène.

**8.** Formulation selon l'une quelconque des revendications 1 à 7, **caractérisée en ce que**, dans la formule (I), B représente la thymine ou l'adénine.

**9.** Formulation selon l'une quelconque des revendications 1 à 8, **caractérisée en ce qu'**on utilise au moins un composé de formule (I) dans laquelle $L_1$, $L_2$ et/ou $R_3$ représentent un groupement chargé négativement pour obtenir une couche lipidique anionique.

**10.** Formulation selon l'une quelconque des revendications 1 à 8, **caractérisée en ce qu'**on utilise au moins un composé de formule (I) dans laquelle $L_1$, $L_2$ et/ou $R_3$ représentent un groupement chargé cationique pour obtenir une couche lipidique cationique.

**11.** Procédé pour la préparation d'une formulation selon l'une quelconque des revendications 1 à 10, **caractérisé en ce qu'**il comprend les étapes suivantes :

a) préparer un mélange d'au moins un composé amphiphile fonctionnel de formule (I) tel que défini dans la revendication 1 et d'un agent thérapeutique,
b) soumettre ledit mélange à des cycles répétés de chauffage et congélation, de manière à obtenir des nanoparticules contenant ledit agent thérapeutique, et
c) récupérer les nanoparticules contenant ledit agent thérapeutique ainsi obtenues,
d) mettre en présence lesdites nanoparticules avec au moins un composé amphiphile fonctionnel de formule (I) tel que défini ci-dessus ayant une polarité différente de celui mis en oeuvre dans l'étape a), et
e) récupérer les nanoparticules multi-compartiments ainsi obtenues.

**12.** Procédé selon la revendication 11, **caractérisé en ce qu'**on utilise un colipide lors des étapes a) et/ou d).

**13.** Procédé selon l'une des revendications 11 ou 12, **caractérisé en ce qu'**il comprend une étape supplémentaire consistant en la formation d'une couche lipidique neutre constituée d'au moins un composé amphiphile fonctionnel de formule (I) telle que définie dans la revendication 1, ledit composé de formule (I) étant neutre, pour constituer la couche lipidique externe.

**14.** Procédé selon l'une quelconque des revendications 11 à 13, **caractérisé en ce qu'**on introduit à l'étape d) un agent thérapeutique différent de celui utilisé à l'étape a).

**15.** Agent de transport, de vectorisation, ou de délivrance intracellulaire d'agents thérapeutiques, en particulier d'agents anti-tumoraux, comprenant une formulation selon l'une quelconque des revendications 1 à 10.

**16.** Formulation selon l'une quelconque des revendications 1 à 10 pour son utilisation dans le traitement de maladies tumorales.

**17.** Composition pharmaceutique comprenant une formulation selon l'une quelconque des revendications 1 à 10 et un véhicule pharmaceutiquement acceptable.

## Patentansprüche

**1.** Formulierung mit mehreren Kammern in der Form eines Nanopartikels mit einem durchschnittlichen Durchmesser von etwa 1 bis 200 nm, das aus einem festen Kern gebildet ist, der ein therapeutisches Mittel enthält, und mit mindestens zwei Lipidschichten mit unterschiedlicher Polarität umgeben ist, die aus amphiphilen funktionellen Molekülen oder Makromolekülen gebildet sind, worin jede Lipidschicht gebildet ist aus mindestens einer funktionellen amphiphilen Verbindung der Formel (I)

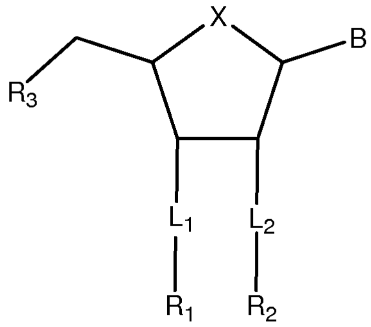

(I)

worin

- X für ein Sauerstoffatom, ein Schwefelatom oder eine Methylengruppe steht,
- B für eine Purin- oder Pyrimidinbase, wie Uracil, Adenin, Guanin, Cytosin, Thymin, Hypoxanthin oder deren Derivate, oder eine mono-oder bicyclische, nicht-natürliche, heterocyclische Base, bei der jeder Ring aus 4 bis 7 Gliedern besteht, gegebenenfalls substituiert, steht;
- $L_1$ und $L_2$, die gleich oder verschieden sind, für Wasserstoff, eine Oxycarbonylgruppe -O-C(O)-, eine Thiocarbamatgruppe -O-C(S)-NH-, eine Carbonatgruppe -O-C(O)-O-, eine Carbamatgruppe -O-C(O)-NH-, ein Sauerstoffatom, eine Phosphatgruppe, eine Phosphonatgruppe oder eine Heteroarylgruppe mit 1 bis 4 Stickstoffatomen, durch eine gesättigte oder ungesättigte, lineare oder verzweigte $C_2$-$C_{30}$-Kohlenwasserstoffkette substituiert oder unsubstituiert, stehen,

oder auch $L_1$ und $L_2$ zusammen eine Ketalgruppe bilden mit der Formel

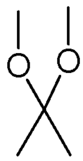

oder auch $L_1$ oder $L_2$ für Wasserstoff steht und das jeweils andere für eine Hydroxygruppe oder eine Heteroarylgruppe mit 1 bis 4 Stickstoffatomen, unsubstituiert oder durch eine lineare oder verzweigte $C_2$-$C_{30}$-Alkylkette substituiert, steht;

- $R_1$ und $R_2$, die gleich oder verschieden sind, stehen für
  - eine lineare oder verzweigte, gesättigte oder teilweise ungesättigte, gegebenenfalls vollständig oder teilweise fluorierte $C_2$-$C_{30}$-, vorzugsweise $C_6$-$C_{25}$-, insbesondere $C_8$-$C_{25}$-Kohlenwasserstoffkette, unsubstituiert oder an dem Kohlenstoffende der Kette durch ein Fluoratom oder durch ein Ester oder ein Benzylether oder Naphthylether substituiert, oder
  - eine Diacylkette, worin jede Acylkette $C_2$-$C_{30}$ ist, oder
  - eine Diacylglycerin-, Sphingosin- oder Ceramidgruppe, oder
  - wenn $L_1$ oder $L_2$ für Wasserstoff steht und das jeweils andere für eine Hydroxygruppe oder eine Heteroarylgruppe mit 1 bis 4 Stickstoffatomen steht, $R_1$ und $R_2$ nicht vorkommen;

$R_3$ steht für

- eine Hydroxy-, Amino-, Phosphat-, Phosphonat-, Phosphatidylcholin-, O-Alkylphosphatidylcholin-, Thiophosphat-, Phosphonium-, $NH_2$-$R_4$-, $NHR_4R_5$- oder $NR_4R_5R_6$-Gruppierung, worin $R_4$, $R_5$ und $R_6$, die gleich oder verschieden sind, für ein Wasserstoffatom oder eine lineare oder verzweigte $C_1$-$C_5$-Alkylkette oder ein lineares oder verzweigtes $C_1$-$C_5$-Hydroxy-alkylen stehen, oder
- eine lineare oder verzweigte $C_2$-$C_{30}$-Alkylkette, gegebenenfalls durch eine Hydroxygruppe substituiert, oder
- einen Cyclodextrinrest, oder
- einen Rest

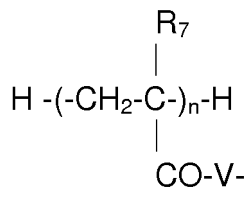

worin V für eine -O-, -S- oder -NH-Bindung steht, $R_7$ für H oder $CH_3$ steht, und n = 1 bis 500, oder eine Gruppe $-(CH_2)_n$-V-$R_8$, worin $R_8$ für ein $C_2$-$C_{30}$-Alkyl steht, und n = 1 bis 500, oder
- eine Heteroarylgruppe mit 1 bis 4 Stickstoffatomen, unsubstituiert oder durch ein $C_2$-$C_{30}$-Alkyl oder durch eine $(CH_2)_m$-O-$(CH_2)_p$-$R_9$-Gruppe substituiert, worin m = 1 bis 6 und p = 0 bis 10 und $R_9$ für eine cyclische Ketalgruppe mit 5 bis 7 Kohlenstoffatomen, unsubstituiert oder durch mindestens ein lineares oder verzweigtes $C_2$-$C_{30}$-Alkyl oder durch einen Sterinrest substituiert, steht, oder auch

- $R_3$ durch eine kovalente Bindung an einen anderen Substituenten $R_3$ gebunden ist, der gleich oder verschieden ist, aus einer anderen Verbindung der Formel (I), die gleich oder verschieden ist, um eine Verbindung der Formel (I) in der Form eines Dimers zu bilden,

und jede Lipidschicht eine andere Polarität als die vorherige aufweist.

2. Formulierung nach Anspruch 1, **dadurch gekennzeichnet, dass** jede Lipidschicht eine Kammer bildet, die geeignet ist, das gleiche oder ein anderes therapeutisches Mittel als das, das im Kern vorhanden ist, aufzunehmen.

3. Formulierung nach einem der Ansprüche 1 oder 2, **dadurch gekennzeichnet, dass** ein Colipid in mindestens einer Lipidschicht vorhanden sein kann.

4. Formulierung nach einem der Ansprüche 1 bis 3, **dadurch gekennzeichnet, dass** das therapeutische Mittel ein Antitumormittel ist.

5. Formulierung nach Anspruch 4, **dadurch gekennzeichnet, dass** das therapeutische Mittel aus Platin- oder Rutheniumkomplexen, die eine Bindung an Platinkomplexe herstellen können, oder auch anorganischen Komplexen ohne Platin auf der Basis von Ruthenium II oder III, Titan, Gallium, Kobalt, Eisen oder Gold ausgewählt ist.

6. Formulierung nach einem der Ansprüche 1 bis 5, **dadurch gekennzeichnet, dass** das therapeutische Mittel aus Cisplatin, Carboplatin, Oxaliplatin, Nedaplatin und Lobaplatin ausgewählt ist.

7. Formulierung nach einem der Ansprüche 1 bis 6, **dadurch gekennzeichnet, dass** in Formel (I) X für Sauerstoff steht.

8. Formulierung nach einem der Ansprüche 1 bis 7, **dadurch gekennzeichnet, dass** in Formel (I) B für Thymin oder Adenin steht.

9. Formulierung nach einem der Ansprüche 1 bis 8, **dadurch gekennzeichnet, dass** zumindest eine Verbindung der Formel (I) verwendet wird, worin $L_1$, $L_2$ und/oder $R_3$ für eine negativ geladene Gruppe stehen, um eine anionische Lipidschicht zu erhalten.

10. Formulierung nach einem der Ansprüche 1 bis 8, **dadurch gekennzeichnet, dass** mindestens eine Verbindung

der Formel (I) verwendet wird, worin $L_1$, $L_2$ und/oder $R_3$ für eine kationisch geladene Gruppe stehen, um eine kationische Lipidschicht zu erhalten.

**11.** Verfahren zur Herstellung einer Formulierung nach einem der Ansprüche 1 bis 10, **dadurch gekennzeichnet, dass** es die folgenden Schritte umfasst:

a) Herstellen einer Mischung aus mindestens einer funktionellen amphiphilen Verbindung der Formel (I) wie sie in Anspruch 1 definiert ist, und einem therapeutischen Mittel,
b) Unterziehen der Mischung wiederholten Zyklen eines Erwärmens und Einfrierens, um Nanopartikel zu erhalten, die das therapeutische Mittel enthalten, und
c) Gewinnen der Nanopartikel, die das so erhaltene therapeutische Mittel enthalten,
d) Zusammenbringen der Nanopartikel mit mindestens einer funktionellen amphiphilen Verbindung der Formel (I), wie sie vorstehend definiert ist, die eine anderen Polarität aufweist als die, die in Schritt a) implementiert ist, und
e) Gewinnen der so erhaltenen Mehrkammer-Nanopartikel.

**12.** Verfahren nach Anspruch 11, **dadurch gekennzeichnet, dass** während der Schritte a) und/oder d) ein Colipid verwendet wird.

**13.** Verfahren nach einem der Ansprüche 11 oder 12, **dadurch gekennzeichnet, dass** es einen weiteren Schritt umfasst, der aus dem Bilden einer neutralen Lipidschicht besteht, die aus mindestens einer funktionellen amphiphilen Verbindung der Formel (I), wie sie in Anspruch 1 definiert ist, besteht, wobei die Verbindung der Formel (I) neutral ist, um die äußere Lipidschicht zu bilden.

**14.** Verfahren nach einem der Ansprüche 11 bis 13, **dadurch gekennzeichnet, dass** in Schritt d) ein anderes therapeutisches Mittel als das, das in Schritt a) verwendet wird, eingeführt wird.

**15.** Transportmittel zur Vektorisierung oder intrazellulären Abgabe von therapeutischen Mitteln, insbesondere Antitumormitteln, das eine Formulierung nach einem der Ansprüche 1 bis 10 umfasst.

**16.** Formulierung nach einem der Ansprüche 1 bis 10 zur Verwendung bei der Behandlung von Tumorerkrankungen.

**17.** Pharmazeutische Zusammensetzung, umfassend eine Formulierung nach einem der Ansprüche 1 bis 10 und einen pharmazeutisch verträglichen Träger.

## Claims

**1.** Formulation with multiple compartments in the form of nanoparticles having an average diameter of about 1 to 200 nm constituted by a solid core containing a therapeutic agent, surrounded by at least two lipid layers of different polarity formed from functional amphiphilic molecules or macromolecules, in which each lipid layer is constituted by at least one functional amphiphilic compound of formula (I)

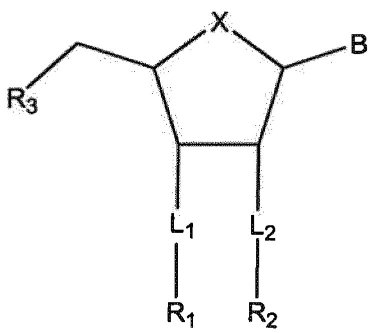

(I)

in which

- X represents an oxygen or sulphur atom or a methylene group,

- B represents purine or pyrimidine base such as uracile, adenine, guanine, cytosine, thymine, hypoxanthine, or their derivatives, or also a non-natural mono-or bi-cyclic heterocyclic base each ring of which comprises 4 to 7 members, optionally substituted;
- $L_1$ and $L_2$, identical or different, represent hydrogen, an oxycarbonyl -O-C(O)-group, a thiocarbamate -O-C(S)-NH- group, a carbonate -O-C(O)-O- group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated, $C_2$-$C_{30}$ hydrocarbon chain,
or also, $L_1$ and $L_2$, together, form a ketal group of formula

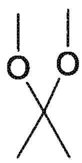

or also $L_1$ or $L_2$ represents hydrogen, and the other represents a hydroxy group or a heteroaryl group comprising 1 to 4 nitrogen atoms, unsubstituted or substituted by a linear or branched $C_2$-$C_{30}$ alkyl chain;
- $R_1$ and $R_2$, identical or different, represent
- a linear or branched $C_2$-$C_{30}$ hydrocarbon chain, preferably $C_6$-$C_{25}$, in particular $C_8$-$C_{25}$, saturated or partially unsaturated, optionally completely or partially fluorinated, unsubstituted or substituted on the carbon at the end of the chain by a fluorine atom or by a benzyl or naphthyl ester or ether, or
- a diacyl chain in which each acyl chain is $C_2$-$C_{30}$, or
- a diacylglycerol, sphingosine or ceramide group, or
- when $L_1$ or $L_2$ represents hydrogen, and the other represents a hydroxy group or a heteroaryl group comprising 1 to 4 nitrogen atoms, $R_1$ and $R_2$ do not exist;
- $R_3$ represents:
- a hydroxy, amino, phosphate, phosphonate, phosphatidylcholine, O-alkyl phosphatidylcholine, thiophosphate, phosphonium, $NH_2$-$R_4$, $NHR_4R_5$ or $NR_4R_5R_6$ group in which $R_4$, $R_5$ and $R_6$, identical or different, represent a hydrogen atom or a linear or branched $C_1$-$C_5$ alkyl or linear or branched $C_1$-$C_5$ hydroxyalkyl chain, or
- a linear or branched $C_2$-$C_{30}$ alkyl chain optionally substituted by a hydroxy group, or
- a cyclodextrin radical, or
- a

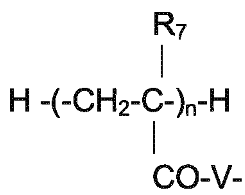

radical in which V represents an -O-,-S-, or -NH- bond, $R_7$ represents H or $CH_3$, and n= 1 to 500,
or a $-(CH_2)_n$-V-$R_8$ group, in which $R_8$ represents a $C_2$-$C_{30}$ alkyl, and n = 1 to 500, or
- a heteroaryl group containing 1 to 4 nitrogen atoms, unsubstituted or substituted by a $C_2$-$C_{30}$ alkyl, or by a $(CH_2)_m$-O-$(CH_2)_p$-$R_9$ group in which m = 1 to 6 and p = 0 to 10 and $R_9$ represents a cyclic ketal group containing 5 to 7 carbon atoms, unsubstituted or substituted by at least one linear or branched $C_2$-$C_{30}$ alkyl or by a sterol radical, or also

- $R_3$ is linked by a covalent bond to another substituent $R_3$, identical or different, of another compound of formula (I), identical or different, in order to form a compound of formula (I) in the form of a dimer,

and each lipid layer has a polarity different from that of the previous one.

2. Formulation according to claim 1, **characterized in that** each lipid layer constitutes a compartment which can comprise a therapeutic agent identical to or different from that present in the core.

3. Formulation according to any one of claims 1 or 2, **characterized in that** a co-lipid can be present in at least one

lipid layer.

**4.** Formulation according to any one of claims 1 to 3, **characterized in that** the therapeutic agent is an anti-neoplastic agent.

**5.** Formulation according to claim 4, **characterized in that** the therapeutic agent is chosen from the platinum complexes or ruthenium capable of binding to platinum complexes, or also the inorganic complexes without platinum based on ruthenium II or III, titanium, gallium, cobalt, iron or gold.

**6.** Formulation according to any one of claims 1 to 5, **characterized in that** the therapeutic agent is chosen from cisplatin, carboplatin, oxaliplatin, nedaplatin and lobaplatin.

**7.** Formulation according to any one of claims 1 to 6, **characterized in that**, in formula (I), X represents oxygen.

**8.** Formulation according to any one of claims 1 to 7, **characterized in that**, in formula (I), B represents thymine or adenine.

**9.** Formulation according to any one of claims 1 to 8, **characterized in that** at least one compound of formula (I) is used, in which $L_1$, $L_2$ and/or $R_3$ represent a negatively charged group in order to obtain an anionic lipid layer.

**10.** Formulation according to any one of claims 1 to 8, **characterized in that** at least one compound of formula (I) is used, in which $L_1$, $L_2$ and/or $R_3$ represent a cationic charged group in order to obtain a cationic lipid layer.

**11.** Method for preparing a formulation according to any one of claims 1 to 10, **characterized in that** it comprises the following steps:

a) preparing a mixture of at least one functional amphiphilic compound of formula (I) as defined in claim 1 and a therapeutic agent,
b) subjecting said mixture to repeated heating and freezing cycles, in order to obtain nanoparticles containing said therapeutic agent, and
c) recovering the nanoparticles containing said therapeutic agent thus obtained,
d) bringing said nanoparticles into the presence of at least one functional amphiphilic compound of formula (I) as defined above, having a polarity different from that utilized in step a), and
e) recovering the multi-compartment nanoparticles thus obtained.

**12.** Method according to claim 11, **characterized in that** a co-lipid is used during steps a) and/or d).

**13.** Method according to one of the claims 11 or 12, **characterized in that** it comprises an additional step consisting of the formation of a neutral lipid layer constituted by at least one functional amphiphilic compound of formula (I) as defined in claim 3, said compound of formula (I) being neutral, to constitute the external lipid layer.

**14.** Method according to any one of claims 11 to 13, **characterized in that** a therapeutic agent different from that used in step a) is introduced in step d).

**15.** Agent for the transport, the targeting or the intracellular delivery of therapeutic agents, in particular of anti-neoplastic agents comprising a formulation according to any one of claims 1 to 10.

**16.** Formulation according to any one of claims 1 to 11 for use in the treatment of tumor diseases.

**17.** Pharmaceutical composition comprising a formulation according to any one of claims 1 to 10 and a pharmaceutically acceptable vehicle.

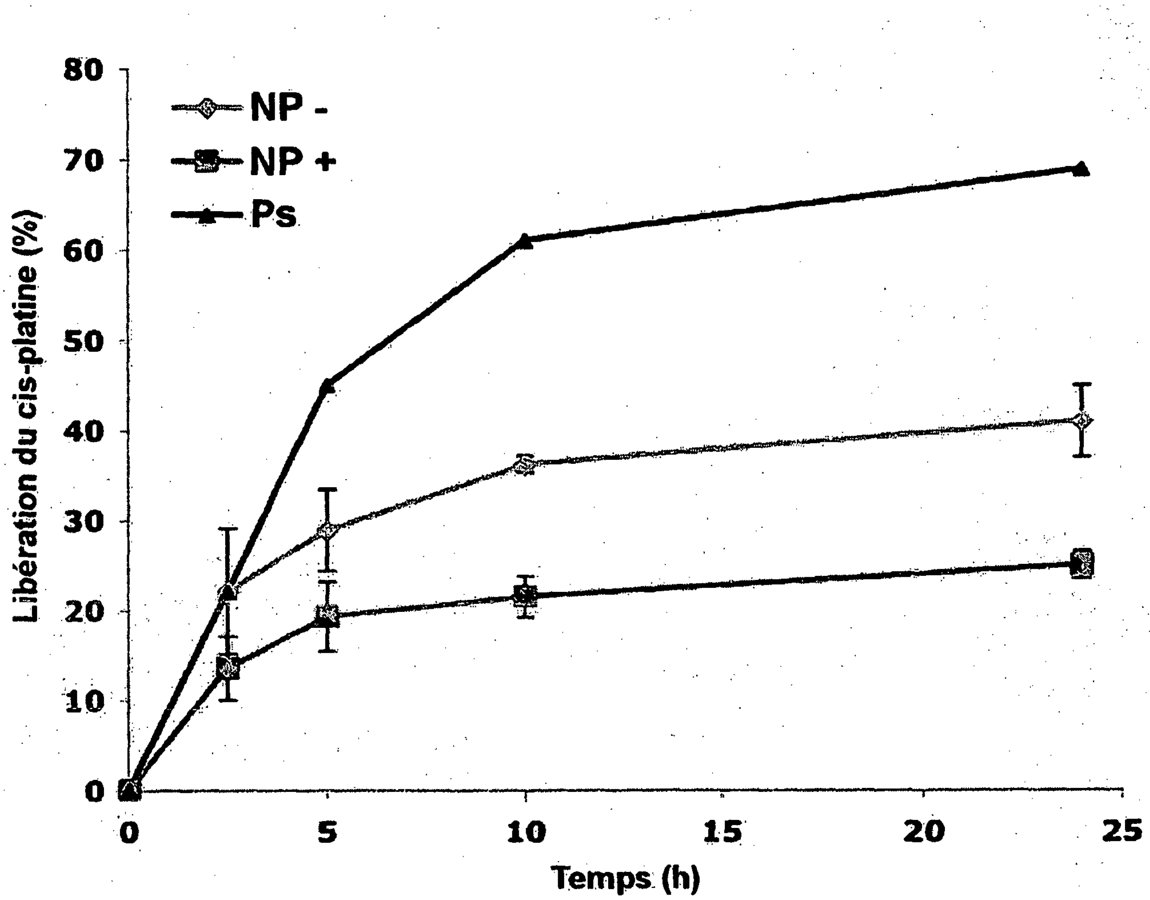
80
70
60
50
40
30
20
10
0
NP -
NP +
Ps
Libération du cis-platine (%)
0
5
10
15
20
25
Temps (h)

Figure 1

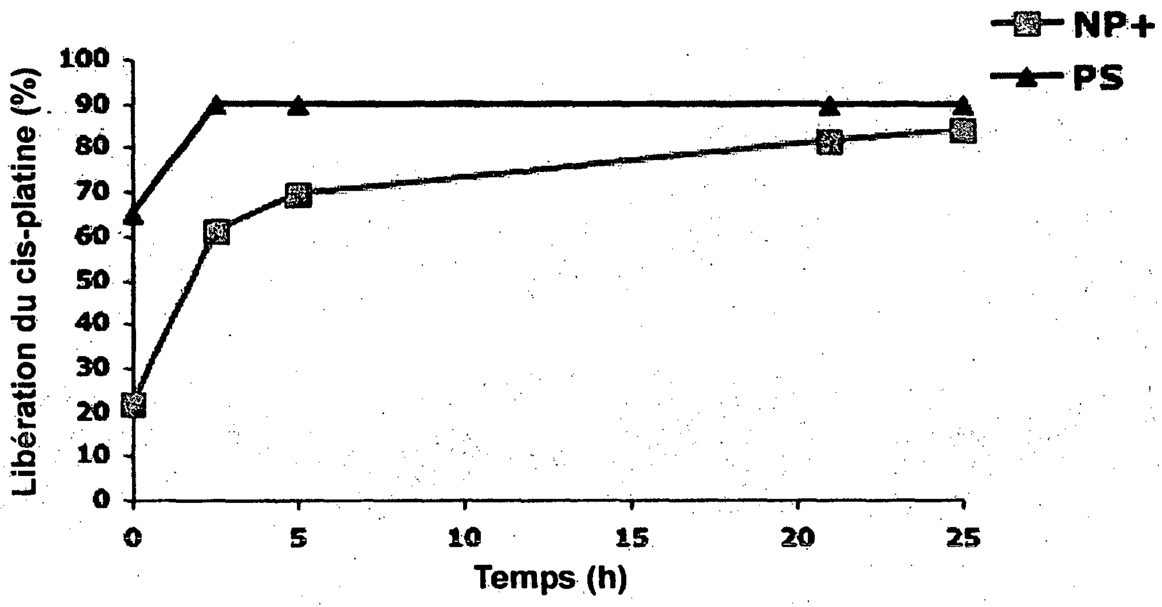
NP+
PS
100
90
80
70
60
50
40
30
20
10
0
Libération du cis-platine (%)
0
5
10
15
20
25
Temps (h)

Figure 2

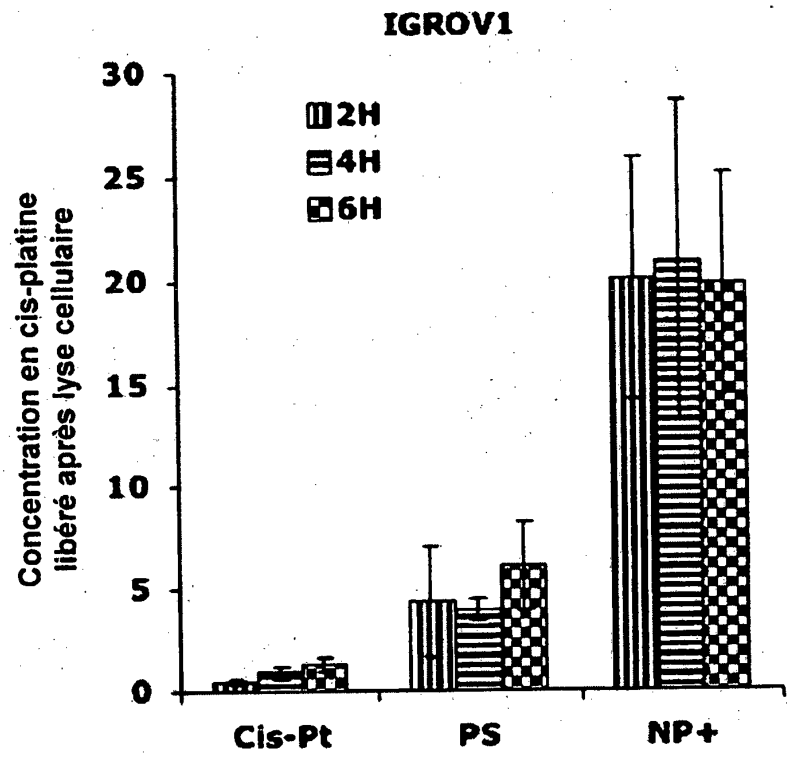


Figure 3A

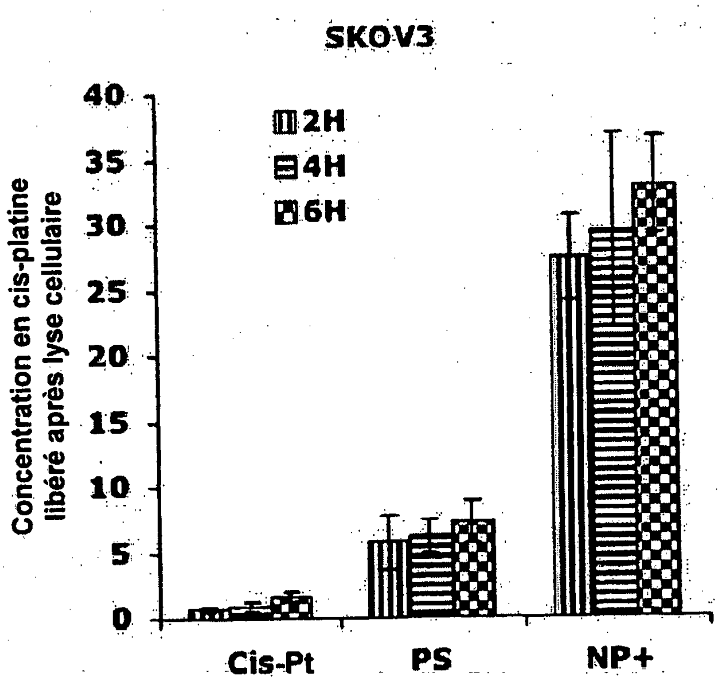


Figure 3B

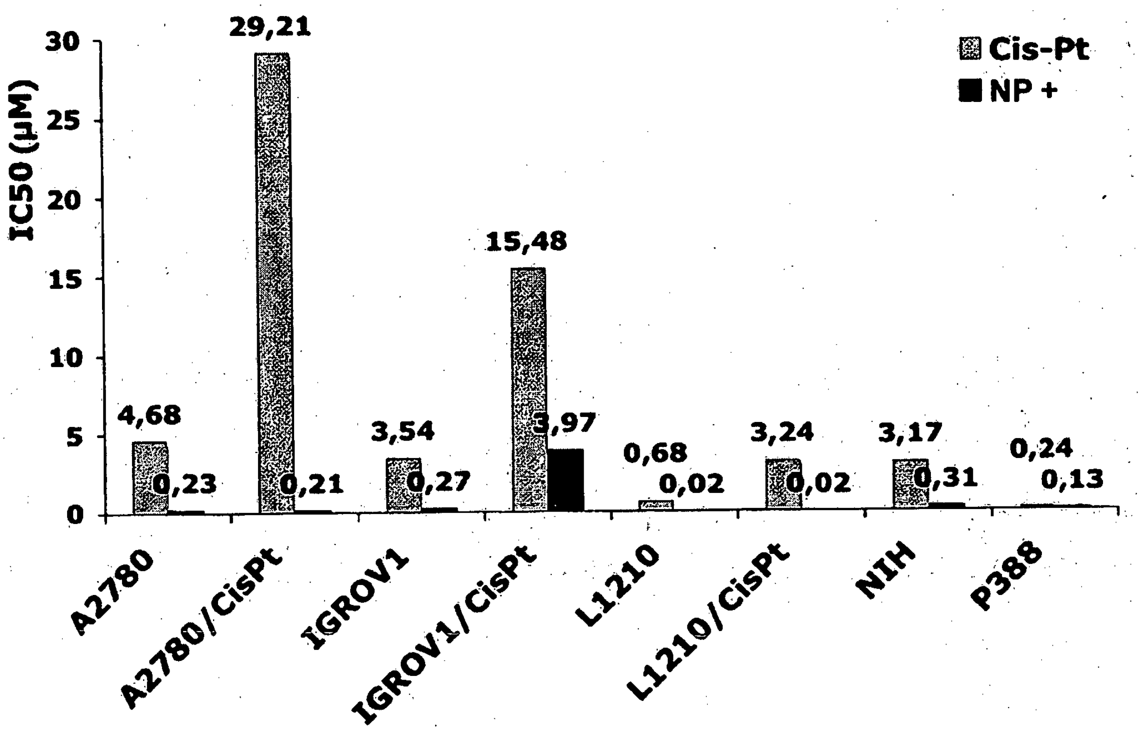
IC50 (µM)
30
25
20
15
10
5
0
29,21
15,48
4,68
3,54
3,97
0,68
3,24
3,17
0,24
0,23
0,21
0,27
0,02
0,02
0,31
0,13
Cis-Pt
NP +
A2780
A2780/CisPt
IGROV1
IGROV1/CisPt
L1210
L1210/CisPt
NIH
P388

Figure 4

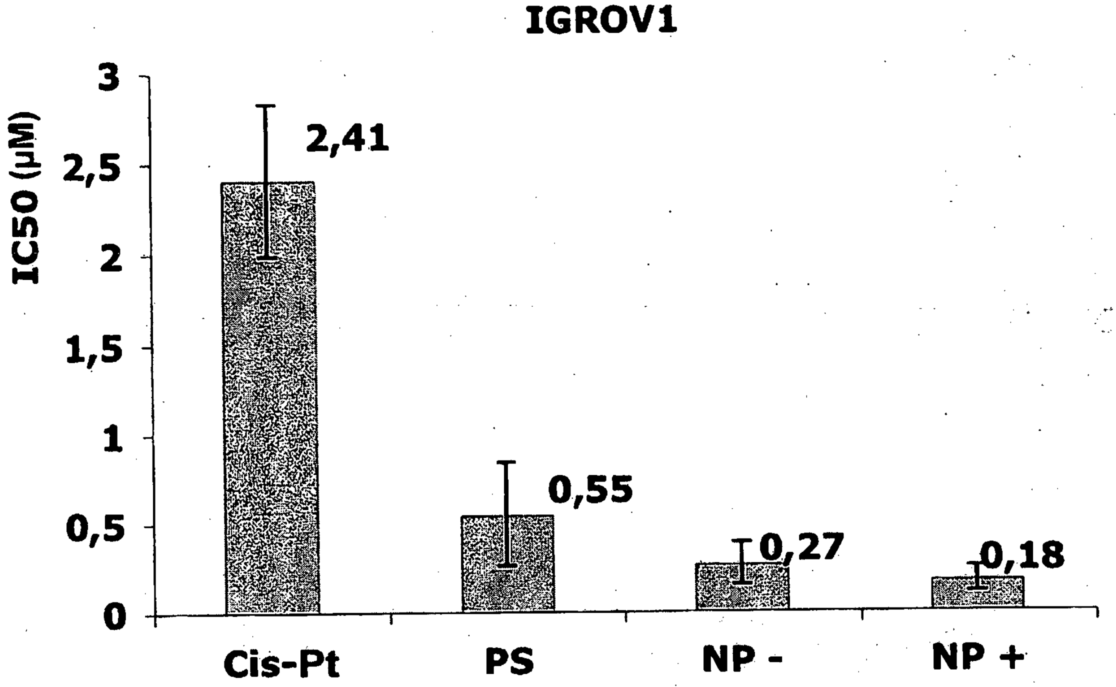


Figure 5A

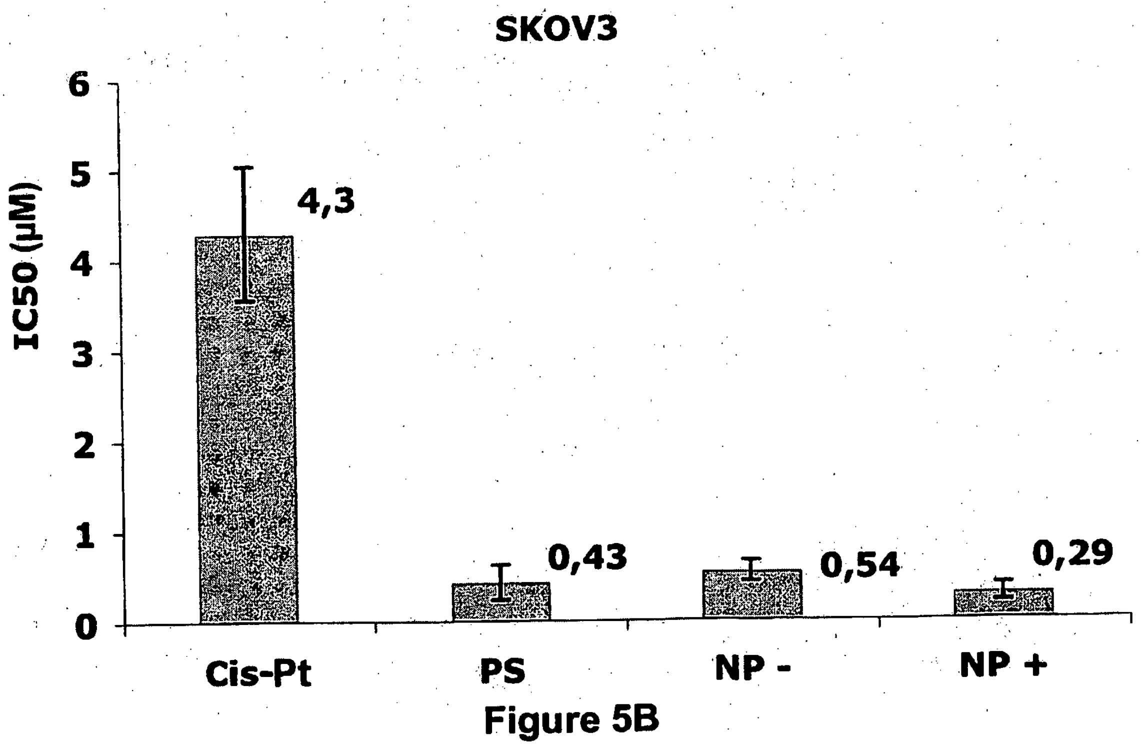


Figure 5B

# Figure 6

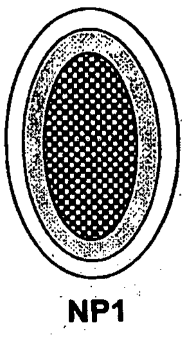

NP1

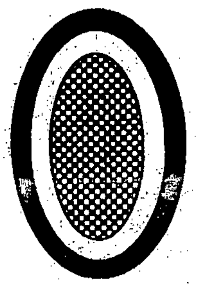

NP2

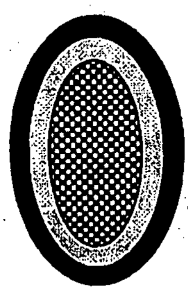

NP3

# Figure 7

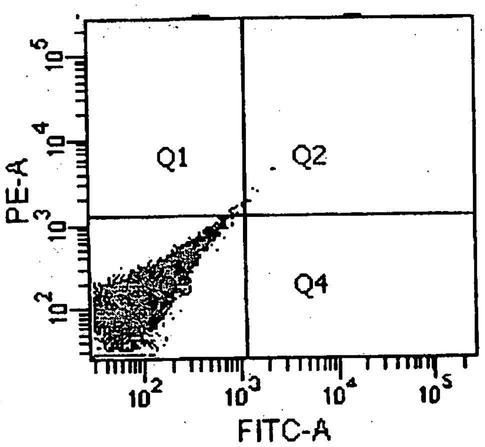


7A

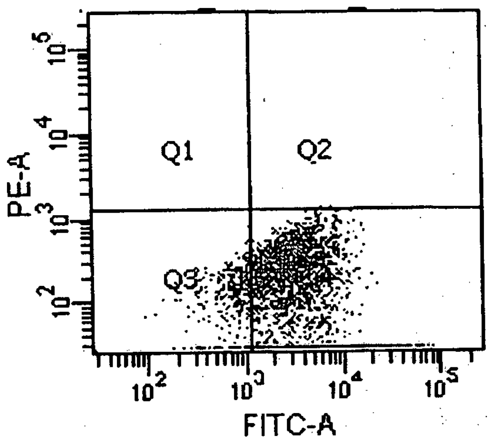


7B

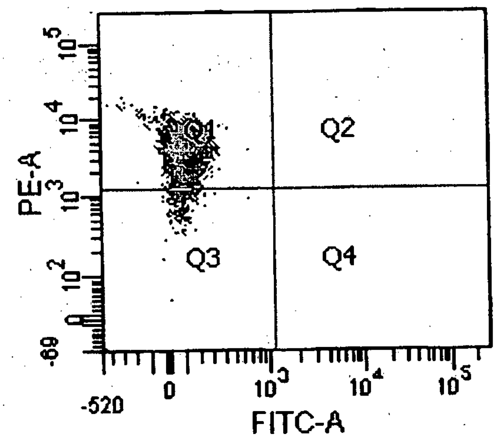


7C

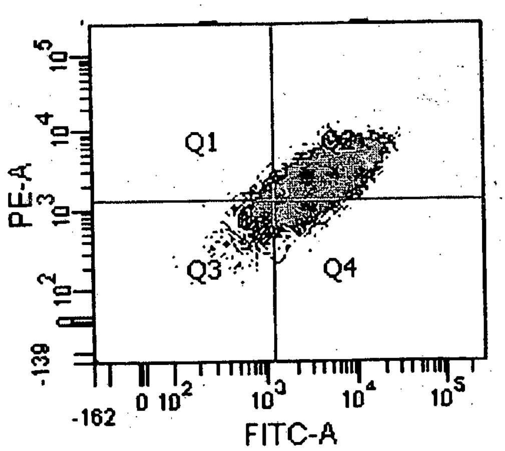


7D

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5178876 A
- US 6001817 A
- US 7908160 B
- WO 0132139 A
- US 2008089836 A
- WO 2009098404 A
- WO 2005116043 A


### Littérature non-brevet citée dans la description


- **ANDREA MAYER et al.** *Toxicology,* 2009, vol. 258, 139-147
- **OTT I. ; GUST R.** *Arch. Pharm. Chem. Life Sci.,* 2007, vol. 340, 117-126
- **REEDIJK J.** *Curr Opin Chem Biol.,* 1999, vol. 3, 236-40
- **HAIMEI CHEN et al.** *J. Am. Chem. Soc.,* 2003, vol. 125, 173-186
- **SCHLAWE D. et al.** *Angew. Chem. Int. Ed.,* 2004, 1731-1734
- **PAULINE CHABAUD et al.** *Bioconjugate Chem.,* 2006, vol. 17, 466-472